# EUROPEAN PATENT APPLICATION

(11) **EP 4 728 969 A1**
(43) Date of publication of application: **22.04.2026**
(21) Application number: 24903891.0
(22) Date of filing: 28.08.2024
(51) Int. Cl.: A61B 5/00, A63B 71/06, A63B 24/00, A61B 5/11, A63B 23/04

(54) **WEARABLE DEVICE, METHOD, AND STORAGE MEDIUM FOR PROVIDING INFORMATION ON PHYSICAL ACTIVITY OF USER**

(30) Priority: 15.12.2023 KR 20230183908; 25.01.2024 KR 20240011794
(71) Applicant: Samsung Electronics Co., Ltd., Suwon-si, Gyeonggi-do 16677 (KR)
(72) Inventor: HAN, Keunsang, Suwon-si Gyeonggi-do 16677 (KR); SON, Jihye, Suwon-si Gyeonggi-do 16677 (KR); JO, Minhwan, Suwon-si Gyeonggi-do 16677 (KR)
(74) Representative: Nederlandsch Octrooibureau
(86) International application number: PCT/KR2024/012914
(87) International publication number: WO 2025/127320

(57) **Abstract**

According to an embodiment, a method performed by a wearable device may comprise an operation of identifying that a user's exercise based on designated operations is started. The method may comprise an operation of acquiring second information of the designated operations. The method may comprise an operation of acquiring second information of an operation of the user's exercise as the designated operations are performed. The method may comprise an operation of acquiring a first value indicating the number of times the designated operations for the second information are performed, on the basis of a first threshold value related to the first information. The method may comprise an operation of acquiring a second value indicating the number of times the designated operations for the second information are performed, on the basis of a second threshold value related to the first information. The method may comprise an operation of displaying the first value and the second value on a display.

## Description

### [Technical Field]

The disclosure relates to a wearable device, a method and a storage medium for providing information on physical activity of a user.

### [Background Art]

Various services are provided through a wearable device. The wearable device may operate while worn on a part of a user's body. While worn on a part of the user's body, the wearable device may identify the user's biometric information and provide a service based on the user's biometric information.

The above-described information may be provided as a related art for the purpose of helping to understand the present disclosure. No claim or determination is raised as to whether any of the above-described information may be applied as a prior art related to the present disclosure.

### [Disclosure]

### [Technical Solution]

According to an embodiment, a wearable device may comprise a display, at least one sensor, a first memory storing first information on exercise movement, a second memory storing instructions, and at least one processor. The instructions, when being executed by the processor, cause the wearable device to identify that an exercise of a user based on designated motions is started. The instructions, when being executed by the processor, cause the wearable device to obtain second information on the designated motions. The instructions, when being executed by the processor, cause the wearable device to obtain, based on a first threshold value related to the first information, a first value indicating the number of times the designated motions with respect to the second information were performed. The instructions, when being executed by the processor, cause the wearable device to obtain, based on a second threshold value related to the first information, a second value indicating the number of times the designated motions with respect to the second information were performed. The instructions, when being executed by the processor, cause the wearable device to display the first value and the second value on the display.

According to an embodiment, a method performed by a wearable device may comprise identifying that an exercise of a user based on designated motions is started. The method may comprise obtaining second information on the designated motions. The method may comprise obtaining second information on exercise movement of the user, in accordance with performance of the designated motions. The method may comprise obtaining, based on a first threshold value related to the first information, a first value indicating the number of times the designated motions with respect to the second information were performed. The method may comprise obtaining, based on a second threshold value related to the first information, a second value indicating the number of times the designated motions with respect to the second information were performed. The method may comprise displaying the first value and the second value on the display.

According to an embodiment, a non-transitory computer readable storage medium may store one or more programs. The one or more programs may comprise instructions which, when being executed by a processor of a wearable device with a display, cause the wearable device to identify that an exercise of a user based on designated motions is started. The one or more programs may comprise instructions which cause the wearable device to obtain second information on the designated motions. The one or more programs may comprise instructions which cause the wearable device to obtain, based on a first threshold value related to the first information, a first value indicating the number of times the designated motions with respect to the second information were performed. The one or more programs may comprise instructions which cause the wearable device to obtain, based on a second threshold value related to the first information, a second value indicating the number of times the designated motions with respect to the second information were performed. The one or more programs may comprise instructions which cause the wearable device to display the first value and the second value on the display.

### [Description of the Drawings]

In relation to the description of the drawings, identical or similar reference numerals may be used for identical or similar components. Further, the above and other aspects, features and advantages of certain embodiments of the present disclosure will be more apparent from the following detailed description, taken in conjunction with the accompanying drawings, in which:
FIG. 1 is a block diagram of an electronic device in a network environment according to various embodiments;
FIGS. 2A and 2B are perspective views illustrating an example electronic device according to various embodiments;
FIG. 3 is an exploded perspective view illustrating an example electronic device according to various embodiments;
FIG. 4A is a diagram illustrating an example of a wearable device for identifying various exercises of a user, according to various embodiments;
FIG. 4B is a graph illustrating an example of a time section for performing a plurality of sets according to a designated motion, according to various embodiments;
FIG. 5 is a block diagram illustrating an example configuration of a wearable device according to various embodiments;
FIG. 6A is a graph illustrating an example operation of a wearable device for identifying the number of times designated motions were performed, according to various embodiments;
FIG. 6B is a graph illustrating an example operation of a wearable device for identifying the number of times designated motions were performed, according to various embodiments;
FIG. 7A is a flowchart illustrating an example operation of a wearable device according to various embodiments;
FIG. 7B is a flowchart illustrating an example operation of a wearable device according to various embodiments;
FIG. 8 is a graph illustrating an example operation of a wearable device according to various embodiments;
FIG. 9 is a graph illustrating an example operation of a wearable device according to various embodiments;
FIG. 10 is a diagram illustrating an example of a screen of a wearable device according to various embodiments;
FIG. 11A is a diagram illustrating an example operation of a wearable device for a squat exercise according to various embodiments;
FIG. 11B is a diagram illustrating an example operation of a wearable device for an arm curl exercise according to various embodiments;
FIG. 12 includes graphs illustrating example operation of a wearable device according to various embodiments;
FIG. 13 is a diagram illustrating an example operation of a wearable device for displaying a result of an exercise of a user, according to various embodiments;
FIG. 14 is a graph illustrating an example operation of a wearable device for displaying a result of an exercise of a user, according to various embodiments; and
FIG. 15 is a flowchart illustrating an example operation of a wearable device according to various embodiments.

### [Mode for Invention]

Hereinafter, embodiments of the present disclosure will be described in detail with reference to the drawings so that those skilled in the art may easily implement the present disclosure. However, the present disclosure may be implemented in several different forms and is not limited to the embodiments described herein. In relation to the description of the drawing, identical or similar reference numerals may be used for identical or similar components. In addition, in the drawings and related descriptions, descriptions of well-known functions and configurations may be omitted for clarity and brevity.

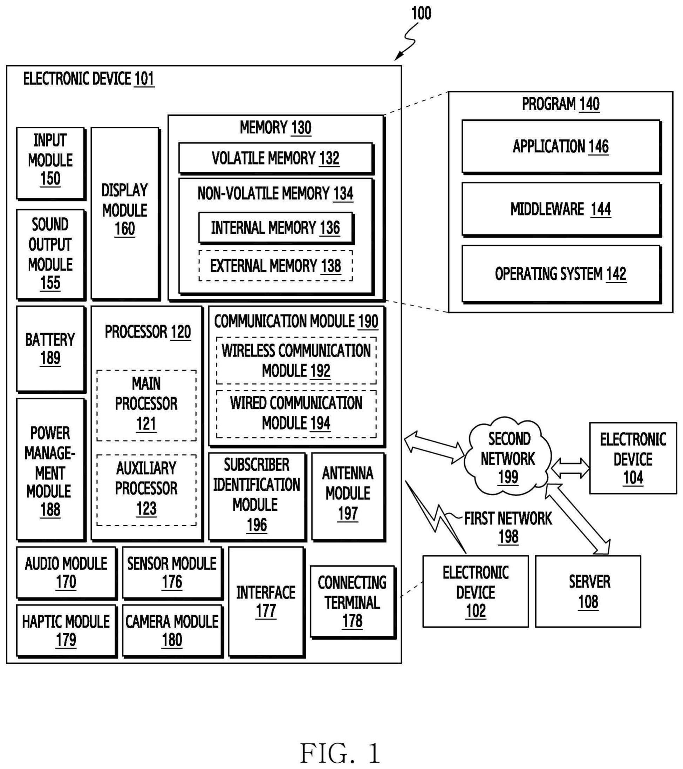

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to an embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, an HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to an embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element including a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, an RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In an embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

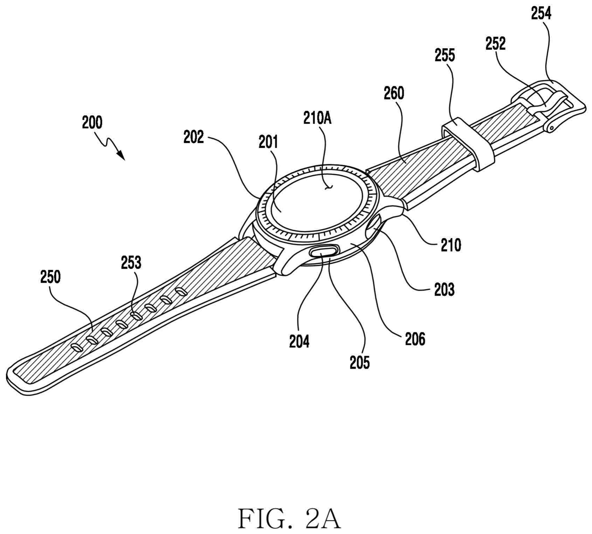

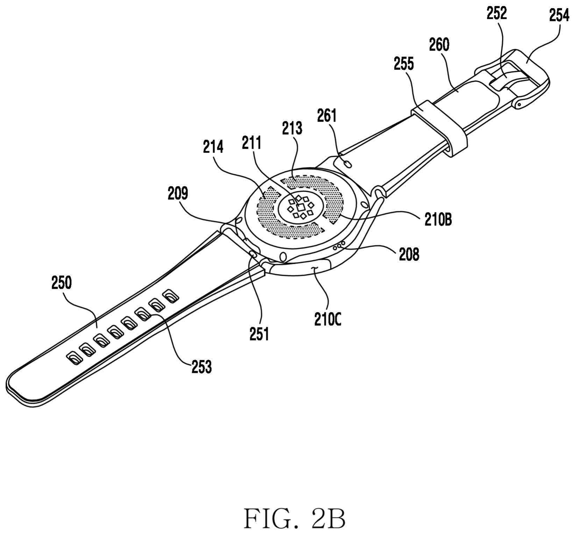

FIGS. 2A and 2B are perspective views illustrating an example electronic device according to various embodiments.

Referring to FIGS. 2A and 2B, according to an embodiment, an electronic device 200 (e.g., the electronic device 101 of FIG. 1) may include a housing 210 including a first surface (or a front surface) 210A, a second surface (or a rear surface) 210B, and a side surface 210C surrounding a space between the first surface 210A and the second surface 210B, and binding members 250 and 260 connected, directly or indirectly, to at least a part of the housing 210 and detachably couple the electronic device 200 to a part of a user's body (e.g., a wrist, an ankle, etc.). In another embodiment (not illustrated), the housing may refer to a structure forming some of the first surface 210A, the second surface 210B, and the side surface 210C of FIGS. 2A and 2B. According to an embodiment, at least a part of the first surface 210A may be formed by a substantially transparent front plate 201 (e.g., a glass plate including various coating layers, or a polymer plate). The second surface 210B may be formed by a substantially opaque rear plate 207. For example, the rear plate 207 may be formed by coating or colored glass, ceramic, polymer, metal (e.g. aluminum, stainless steel (STS), or magnesium), or a combination of at least two of the materials. The side surface 210C may be formed by a side bezel structure (or "side member") 206 coupled, directly or indirectly, to the front plate 201 and the rear plate 207 and including a metal and/or a polymer. In some embodiments, the rear plate 207 and the side bezel structure 206 may be integrally formed and may include the same material (e.g., a metal material such as aluminum). The binding members 250 and 260 may be formed of various materials and shapes. An integral unit link and a plurality of unit links may be formed to flow with each other by a woven fabric, leather, rubber, urethane, metal, ceramic, or a combination of at least two of the materials.

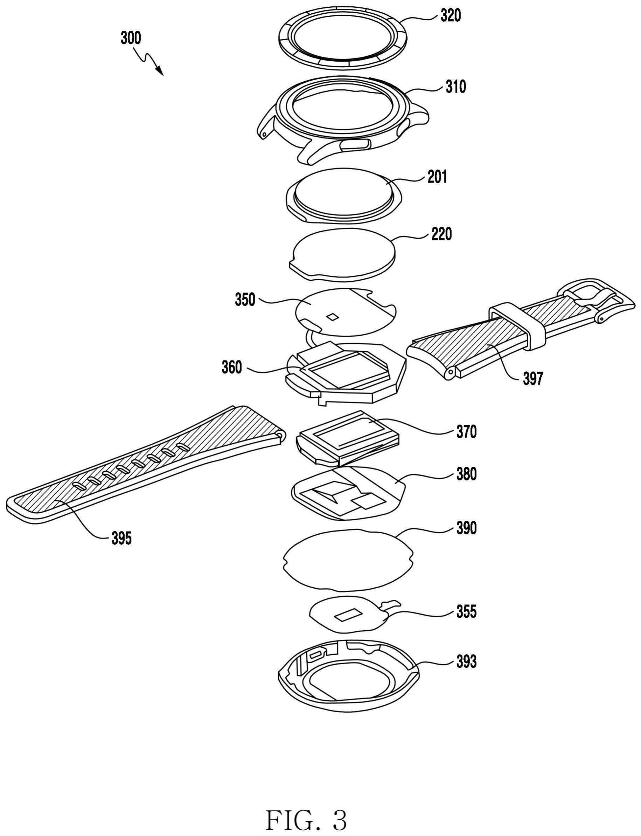

According to an embodiment, the electronic device 200 may include at least one of a display 220 (refer to FIG. 3), audio modules 205 and 208, a sensor module 211 comprising at least one sensor, key input devices 202, 203 and 204, and a connector hole 209. In some embodiments, the electronic device 200 may omit at least one (e.g., key the input devices 202, 203 and 204, the connector hole 209, or the sensor module 211) of the components or may additionally include another component.

The display 220 may be visually exposed, for example, through a substantial part of the front plate 201. The shape of the display 220 may be a shape corresponding to the shape of the front plate 201, and may have various shapes such as a circle, an ellipse, or a polygon. The display 220 may be coupled to, or disposed adjacent to, a touch detecting circuit, a pressure sensor capable of measuring the intensity (pressure) of a touch, and/or a fingerprint sensor.

The audio modules 205 and 208 may include a microphone hole 205 and a speaker hole 208. In the microphone hole 205, a microphone for obtaining an external sound may be disposed inside, and in some embodiments, a plurality of microphones may be disposed to detect the direction of the sound. The speaker hole 208 may be used as an external speaker and a receiver for calls. In some embodiments, the speaker hole 208 and the microphone hole 205 may be implemented as one hole, or a speaker may be included without the speaker hole 208 (e.g., a piezo speaker).

The sensor module 211 may generate an electrical signal or data value corresponding to an internal operating state of the electronic device 200 or an external environmental state. The sensor module 211 may include, for example, a biometric sensor module 211 (e.g., an HRM sensor) disposed on the second surface 210B of the housing 210. The electronic device 200 may further include at least one of a sensor module not illustrated, for example, a gesture sensor, a gyro sensor, an atmospheric pressure sensor, a magnetic sensor, an acceleration sensor, a grip sensor, a color sensor, an infrared (IR) sensor, a biometric sensor, a temperature sensor, a humidity sensor, or an illumination sensor.

The sensor module 211 may include electrode regions 213 and 214 forming a part of the surface of the electronic device 200 and a bio-signal detection circuit (not illustrated) electrically connected, directly or indirectly, to the electrode regions 213 and 214. For example, the electrode regions 213 and 214 may include a first electrode region 213 and a second electrode region 214 disposed on the second surface 210B of the housing 210. The sensor module 211 may be configured such that the electrode regions 213 and 214 obtain an electrical signal from a part of the user's body, and a bio-signal detection circuit detects bio-information of the user based on the electrical signal.

The key input devices 202, 203, and 204 may include a wheel key 202 disposed on the first surface 210A of the housing 210 and rotatable in at least one direction, and/or side key buttons 203 and 204 disposed on the side surface 210C of the housing 210. The wheel key may have a shape corresponding to the shape of the front plate 201. In another embodiment, the electronic device 200 may not include some or all of the above-described key input devices 202, 203, and 204, and the not included key input devices 202, 203, and 204 may be implemented in other forms such as a soft key on the display 220. The connector hole 209 may accommodate connectors (e.g., USB connectors) for transmitting and receiving power and/or data to and from external electronic devices, and include another connector hole (not illustrated) capable of accommodating a connector for transmitting and receiving audio signals to and from an external electronic device. For example, the electronic device 200 may further include a connector cover (not illustrated) that covers at least a part of the connector hole 209 and blocks the inflow of external foreign materials into the connector hole.

The binding members 250 and 260 may be detachably attached to at least a part of the housing 210 using locking members 251 and 261. The binding members 250 and 260 may include one or more of a fixing member 252, a fixing member fastening hole 253, a band guide member 254, and a band fixing ring 255.

The fixing member 252 may be configured to fix the housing 210 and the binding members 250 and 260 to a part of the user's body (e.g., a wrist, an ankle, etc.). Corresponding to the fixing member 252, the fixing member fastening hole 253 may fix the housing 210 and the binding members 250 and 260 to a part of the user's body. The band guide member 254 may be configured to limit a movement range of the fixing member 252 when the fixing member 252 is fastened to the fixing member fastening hole 253, and thus the binding members 250 and 260 may be closely coupled to a part of the user's body. In a state in which the fixing member 252 and the fixing member fastening hole 253 are fastened, the band fixing ring 255 may limit the movement range of the binding members 250 and 260.

FIG. 3 is an exploded perspective view illustrating an example electronic device according to various embodiments.

Referring to FIG. 3, the electronic device 300 (e.g., the electronic device 101 of FIG. 1 or the electronic device 200 of FIGS. 2A to 2B) may include a side bezel structure 310, a wheel key 320, a front plate 201, a display 220, a first antenna 350, a second antenna 355, and a support member 360 (e.g., a bracket), a battery 370, a printed circuit board 380, a sealing member 390, a rear plate 393, and binding members 395 and 397. At least one of the components of the electronic device 300 may be the same as, or similar to, at least one of the components of the electronic device of FIGS. 1 and/or 2A to 2B, and a repeated description thereof will be omitted. The support member 360 may be disposed inside the electronic device 300 to be connected, directly or indirectly, to the side bezel structure 310 or may be integrally formed with the side bezel structure 310. The support member 360 may be formed of, for example, a metal material and/or a non-metal (e.g., a polymer) material. In the support member 360, the display 220 may be coupled, directly or indirectly, to one surface and the printed circuit board 380 may be coupled to the other surface. A processor, a memory, and/or an interface may be mounted on the printed circuit board 380. The processor may include, for example, one or more of a central processing unit, a graphic processing unit (GPU), an application processor, a sensor processor, or a communication processor.

The memory may include, for example, a volatile memory or a nonvolatile memory. The interface may include, for example, a high definition multimedia interface (HDMI), a universal serial bus (USB) interface, an SD card interface, and/or an audio interface. For example, the interface may electrically or physically connect the electronic device 300 to an external electronic device, and may include a USB connector, an SD card/MMC connector, or an audio connector.

The battery 370 is a device for supplying power to at least one component of the electronic device 200/300, and may include, for example, a non-rechargeable primary battery, a rechargeable secondary battery, or a fuel battery. At least a part of the battery 370 may be disposed on substantially the same plane as, for example, the printed circuit board 380. The battery 370 may be integrally disposed inside the electronic device 200/300 or may be detachably disposed from the electronic device 200/300.

The first antenna 350 may be disposed between the display 220 and the support member 360. The first antenna 350 may include, for example, a near field communication (NFC) antenna, a wireless charging antenna, and/or a magnetic secure transmission (MST) antenna. For example, the first antenna 350 may perform short-range communication with an external device, wirelessly transmit and receive power required for charging, and may transmit a short-range communication signal or a self-based signal including payment data. In another embodiment, an antenna structure may be formed by the side bezel structure 310 and/or a part of the support member 360 or a combination thereof.

The second antenna 355 may be disposed between the printed circuit board 380 and the rear plate 393. For example, the second antenna 355 may include a near field communication (NFC) antenna, a wireless charging antenna, and/or a magnetic secure transmission (MST) antenna. For example, the second antenna 355 may perform short-range communication with an external device, wirelessly transmit and receive power required for charging, and may transmit a short-range communication signal or a self-based signal including payment data. In another embodiment, an antenna structure may be formed by the side bezel structure 310 and/or a part of the rear plate 393 or a combination thereof.

The sealing member 390 may be positioned between the side bezel structure 310 and the rear plate 393. The sealing member 390 may be configured to block moisture and foreign substances from flowing into the space surrounded by the side bezel structure 310 and the rear plate 393 from the outside.

According to an embodiment, a wearable device (e.g., the electronic device 101 of FIG. 1, the electronic device 200 illustrated in FIGS. 2A and 2B, or the electronic device 300 illustrated in FIG. 3) may operate while worn by a user. For example, the wearable device may be worn on a part (e.g., wrist, finger, or face) of a user's body. According to an embodiment, the wearable device may be used to provide information on physical activity of a user of the wearable device. For example, the wearable device may provide information on the user's workout (or exercise). According to the user's exercise based on designated motions, the wearable device may provide the user with the number of times the designated motions were performed. Since exercise performance, posture, and/or physical characteristics are different for each user, the wearable device may differently set a threshold value for identifying the number of times designated motions were performed for each user. In the disclosure, technical features for personalizing the threshold value for identifying the number of times designated motions were performed will be described in greater detail.

An operation of the wearable device according to various embodiments may be described below. A wearable device (e.g., a wearable device 400) described below may correspond to the electronic device 101 of FIG. 1, the electronic device 200 of FIGS. 2A and 2B, and/or the electronic device 300 of FIG. 3.

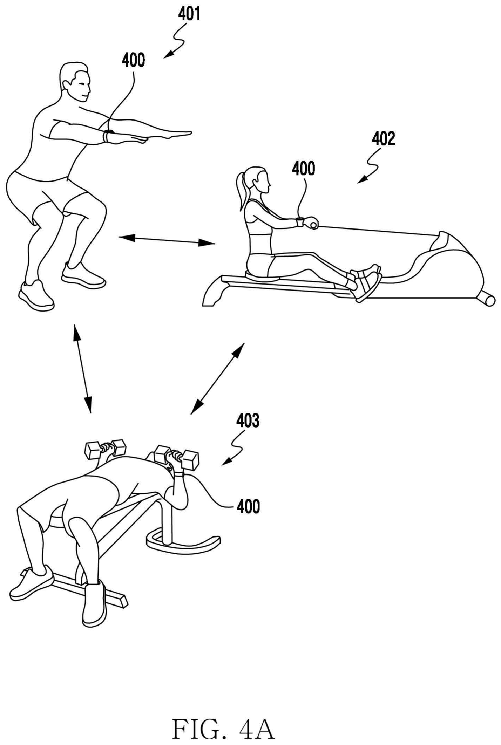

FIG. 4A is a diagram illustrating an example of a wearable device for identifying various exercises of a user, according to various embodiments.

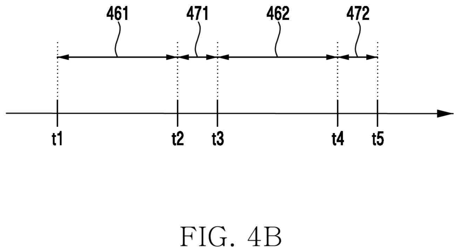

FIG. 4B is a graph illustrating an example of a time section for performing a plurality of sets according to a designated motion, according to various embodiments.

Referring to FIG. 4A, a wearable device 400 may operate while being worn by a user. For example, the wearable device 400 may correspond to the electronic device 200 of FIGS. 2A and 2B and/or the electronic device 300 of FIG. 3. For example, the wearable device 400 may operate while worn on a user's wrist.

FIG. 4A illustrates an example in which the wearable device 400 is worn on a user's wrist, but the disclosure is not limited thereto. The wearable device 400 may operate while worn on a part of the user's body. For example, the wearable device 400 may be worn on one of the user's head, the user's finger, the user's neck, the user's ankle, and the user's ear (or earhole).

According to an embodiment, the wearable device 400 may identify various exercises. For example, while the wearable device 400 is worn on the user's wrist, the wearable device 400 may identify that one of a squat exercise 401, a rowing machine exercise 402, and a dumbbell press exercise 403 is performed. The various exercises illustrated in FIG. 4A are examples and disclosure is not limited thereto.

According to an embodiment, various exercises may be performed by repeatedly performing designated motions. For example, in the squat exercise 401, the user may repeatedly perform a motion of bending and straightening the knee. For example, in the rowing machine exercise 402, the user may repeatedly perform, through a rowing machine, a motion of pulling and releasing a cable of the rowing machine. For example, in the dumbbell press exercise 403, the user may repeatedly perform a motion of raising and lowering a dumbbell using arms.

According to an embodiment, the wearable device 400 may identify the number of times designated motions were performed. For example, the wearable device 400 may identify the number of times the designated motions were performed, using at least one sensor. For example, in the squat exercise 401, the motion of bending and straightening the knee (hereinafter referred to as squat motion) may be counted as 1 time. For example, in the rowing machine 402 exercise, the motion of pulling and releasing the cable of the rowing machine may be counted as 1 time. For example, in the dumbbell press exercise 403, the motion of raising and lowering the dumbbell may be counted as 1 time.

For example, a plurality of sets may be set based on repetition according to a designated value of the designated motion. For example, a designated motion that is repeated for a designated value may form a set. The wearable device 400 may identify that one of the plurality of sets were performed, based on identifying that the number of times the designated motion was performed corresponds to the designated value. As an example, in the squat exercise 401, squat motions repeatedly performed for a designated value may form one set.

For example, the designated value may be set by the user. The user may set repetitions (reps) of the designated motion for forming one set using the wearable device 400. For example, in the squat exercise 401, one set may be set to perform 15 squat motions. The wearable device 400 may identify that one set was performed, based on identifying that the user performed 15 squat motions.

Referring to FIG. 4B, a first set may be performed at a time section 461 between a time t1 and a time t2. For example, the wearable device 400 may identify that a user's exercise based on designated motions starts at the time t1. At the time t2, the wearable device 400 may identify that the number of times the designated motions are performed corresponds to a designated value. The wearable device 400 may identify that the first set is completed based on identifying that the number of times the designated motions are performed corresponds to the designated value. For example, the wearable device 400 may provide the user with a notification indicating that the first set is completed. The notification indicating that the first set is completed may be provided based on at least one of a change in the screen, a sound, and/or a vibration.

According to an embodiment, one set may be set based on a designated time section (e.g., 3 minutes) rather than the number of times designated motions were performed. For example, a time section 461 to perform a first set (or a time section 462 to perform a second set) may be set to a designated time section (e.g., 3 minutes).

A time section 471 between the time t2 and a time t3 may be set as a rest time between the first set and the second set. For example, rest times may be formed between consecutive sets. A break time may be set by the user or predetermined. According to an embodiment, the break time may be maintained until a state of the user is identified as a designated state. For example, the break time may be maintained until the user's heart rate is identified as a designated heart rate. For example, the wearable device 400 may provide a user with a notification indicating that the break time has ended at the time t3. The notification indicating that the break time has ended may be provided based on at least one of a change in a screen, a sound, and/or a vibration.

The second set may be performed at a time section 462 between the time t3 and a time t4. At time t4, the wearable device 400 may identify that the number of times designated motions were performed corresponds to a designated value. The wearable device 400 may identify that the second set is completed based on identifying that the number of times the designated motions were performed corresponds to the designated value. For example, the wearable device 400 may provide a notification indicating that the second set is completed to the user. The notification indicating that the second set is completed may be provided based on at least one of a change in the screen, a sound, and/or a vibration.

A time section 472 between the time t4 and a time t5 may be set as a rest time between the second set and a third set.

As described above, while a user performs an exercise according to a plurality of sets, the wearable device 400 may provide information on the user's exercise. For example, the wearable device 400 may count the number of times designated motions were performed, and display the number of times the designated motions were performed through a display of the wearable device 400.

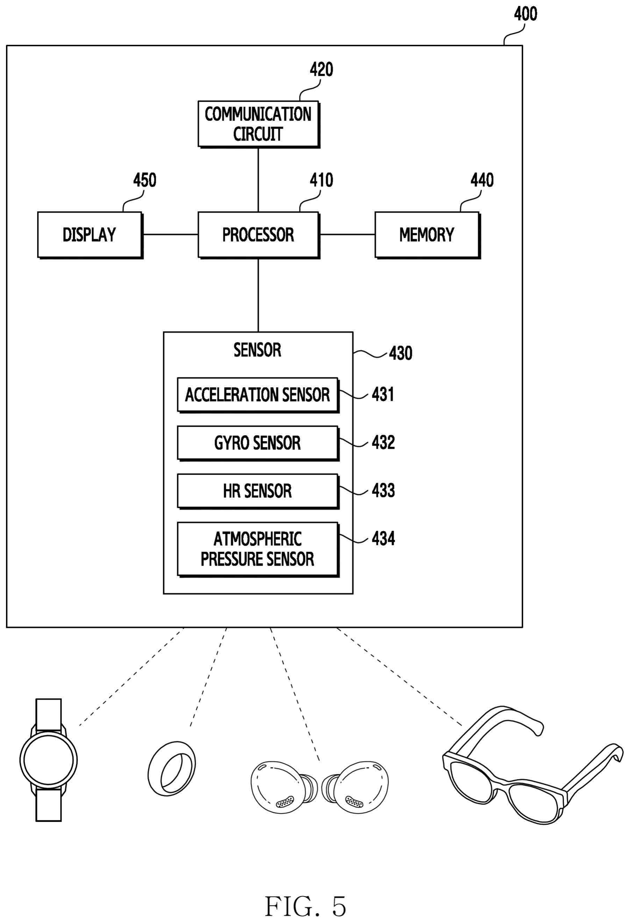

FIG. 5 is a block diagram illustrating an example configuration of a wearable device according to various embodiments.

Referring to FIG. 5, the wearable device 400 may be implemented in various forms. For example, the wearable device 400 may be implemented in various forms capable of being worn by a user, such as a smart watch, a smart band, a smart ring, a wireless earphone, or a smart glass. For example, the wearable device 400 may correspond to the electronic device 101 of FIG. 1, the electronic device 200 of FIGS. 2A and 2B, and/or the electronic device 300 of FIG. 3.

According to an embodiment, the wearable device 400 may operate in connection with an electronic device connected to the wearable device 400. For example, at least a portion of operations of the wearable device 400 described below may be performed in the electronic device connected to the wearable device 400.

According to an embodiment, the wearable device 400 may include a processor (e.g., including processing circuitry) 410, a communication circuit 420, a sensor 430, a memory 440, and/or a display 450. According to an embodiment, the wearable device 400 may include at least one of the processor 410, the communication circuit 420, the sensor 430, the memory 440, and the display 450. For example, at least a portion of the processor 410, the communication circuit 420, the sensor 430, the memory 440, and the display 450 may be omitted according to an embodiment.

According to an embodiment, the processor 410 may correspond to the processor 120 of FIG. 1. The processor 410 may be operably coupled or connected with the communication circuit 420, the sensor 430, the memory 440, and the display 450. For example, the processor 410 being operably coupled with another component may refer, for example, to the processor 410 being capable of controlling the other component. The processor 410 may control the communication circuit 420, the sensor 430, the memory 440, and the display 450. For example, the processor 410 may determine an operation time point of the sensor 430. The processor 410 may control an operation of the sensor 430. The processor 410 may activate or deactivate the sensor 430. The processor 410 may process information obtained from the sensor 430.

According to an embodiment, the processor 410 may be configured with at least one processor. The processor 410 may include at least one processor. According to an embodiment, the processor 410 may include a hardware component for processing data based on one or more instructions. For example, the hardware component for processing data may include an arithmetic and logic unit (ALU), a field programmable gate array (FPGA) and/or a central processing unit (CPU). The processor 410 may include various processing circuitry and/or multiple processors. For example, as used herein, including the claims, the term "processor" may include various processing circuitry, including at least one processor, wherein one or more of at least one processor, individually and/or collectively in a distributed manner, may be configured to perform various functions described herein. As used herein, when "a processor", "at least one processor", and "one or more processors" are described as being configured to perform numerous functions, these terms cover situations, for example and without limitation, in which one processor performs some of recited functions and another processor(s) performs other of recited functions, and also situations in which a single processor may perform all recited functions. Additionally, the at least one processor may include a combination of processors performing various of the recited /disclosed functions, e.g., in a distributed manner. At least one processor may execute program instructions to achieve or perform various functions.

According to an embodiment, the wearable device 400 may include the communication circuit 420. For example, the communication circuit 420 may correspond to at least a portion of the communication module 190 of FIG. 1.

For example, the communication circuit 420 may be used for various radio access technologies (RATs). For example, the communication circuit 420 may be used to perform Bluetooth communication, wireless local area network (WLAN) communication, or ultra wideband (UWB) communication. For example, the communication circuit 420 may be used to perform cellular communication.

For example, the processor 410 may establish a connection with an external electronic device (e.g., the electronic device 102, the electronic device 104, or the server 108 of FIG. 1) through the communication circuit 420. For example, the processor 410 may establish a connection with a server through the communication circuit 420.

According to an embodiment, the wearable device 400 may include the sensor 430. The sensor 430 may be used to obtain various external information. For example, the sensor 430 may be used to obtain data on a user's body. As an example, the sensor 430 may be used to obtain data on the user's state, data on the user's movement, and/or data on the user's heart rate. For example, the sensor 430 may correspond to the sensor module 176 of FIG. 1.

According to an embodiment, the sensor 430 may comprise at least one sensor. For example, the sensor 430 may include at least one of an acceleration sensor 431, a gyro sensor 432, an HR (heart rate) sensor 433, and/or an atmospheric pressure sensor 434.

For example, the acceleration sensor 431 may identify (or measure or detect) acceleration of the wearable device 400 in three directions of x-axis, y-axis, and z-axis. For example, the gyro sensor 432 may identify (or measure or detect) the angular velocity of the wearable device 400 in three directions of the x-axis, the y-axis, and the z-axis. According to an embodiment, the wearable device 400 may include an inertial sensor configured with the acceleration sensor 431 and the gyro sensor 432.

For example, the sensor 430 may include a heart rate (HR) sensor 433 (or a heart rate variability (HRV) sensor). The processor 410 may measure regularity or variability of the heart rate through the HR sensor 433. The processor 410 may obtain information on the regularity or variability of the heart rate through the HR sensor 433.

For example, the atmospheric pressure sensor 434 may identify (or measure or detect) atmospheric pressure around the wearable device 400. The processor 410 may identify a height (or altitude) at which the wearable device 400 is located from the ground, based on data related to the atmospheric pressure around the wearable device 400 identified using the atmospheric pressure sensor 434.

Although not illustrated, the sensor 430 may further include a sensor for obtaining (or identifying, measuring, or detecting) various data related to a user.

For example, the sensor 430 may include a body temperature sensor. The processor 410 may measure a skin temperature of a part of the user's body through the body temperature sensor. The processor 410 may obtain a body temperature of the user, based on the skin temperature of the part of the user's body.

For example, the sensor 430 may include a photoplethysmography (PPG) sensor. The PPG sensor may be used to measure a pulse (or a change in the amount of blood in blood vessels) by identifying the changed amount of photosensitive light according to a change in the volume of the blood vessel. For example, the PPG sensors may be used to identify information on a change in the user's heart rate, information on the user's stress based on the HRV, information on the user's sleep stage, information on the user's respiratory rate, and information on the user's blood pressure.

For example, the sensor 430 may include a blood glucose sensor. The processor 410 may identify the user's blood glucose level, by identifying (or measuring) a current generated by an electro-chemical reaction with the blood glucose in the blood.

According to an embodiment, the wearable device 400 may include the memory 440. The memory 440 may be used to store information or data. For example, the memory 440 may be used to store data obtained from a user. For example, the memory 440 may correspond to the memory 130 of FIG. 1. For example, the memory 440 may be a volatile memory unit or volatile memory units. For example, the memory 440 may be a nonvolatile memory unit or nonvolatile memory units. For example, the memory 440 may be another type of computer-readable medium, such as a magnetic or optical disk. For example, the memory 440 may store data obtained based on an operation (e.g., an algorithm execution operation) performed in the processor 410. For example, the memory 440 may store data (e.g., data on the user's heart rate) obtained from the sensor 430.

For example, the memory 440 may include a first memory and a second memory. The first memory may store first information on exercise movement. The first information on the exercise movement may include at least one threshold value for counting the number of times of the user's exercise. The second memory may store instructions. The instructions, when executed by the processor 410, may cause the wearable device 400 to perform a designated motion.

For example, the display 450 may be used to display various screens. The display 450 may be controlled by the processor 410 including a circuit such as a graphic processing unit (GPU), and output visualized information to the user. The display 450 may be used to output a content, data, or a signal through a screen. For example, the display 450 may correspond to the display module 160 of FIG. 1.

Although not illustrated, the wearable device 400 may further include a speaker, a microphone, and/or a key button. For example, the processor 410 may provide a notification to the user using the speaker. The notifications may include a notification for a start of a set, a notification for completion (or end) of the set, a notification for a start of a break time, a notification for an end of the break time, and/or a notification for a posture for a designated motion. For example, the processor 410 may display the number of times designated motions are identified using the display 450, and receive an input for changing the number of times using at least one of the display 450, a key button, and a microphone. For example, the processor 410 may receive an input indicating completion of a set, using at least one of the display 450, the key button, and the microphone.

According to an embodiment, the processor 410 may be configured to perform at least one algorithm. For example, the at least one algorithm (or at least one instruction) may be stored in the memory 440.

For example, the processor 410 may perform an algorithm for identifying the number of times designated motions were performed. For example, the processor 410 may identify whether a designated motion is performed, using a designated threshold value. The processor 410 may identify whether the designated motion is performed, by comparing a sensor value identified through the sensor 430 with the designated threshold value.

For example, the processor 410 may perform an algorithm for changing the number of times the designated motions were performed. The processor 410 may provide the user with a suggested value for modifying (or changing) the number of times the designated motions were performed. The processor 410 may set (or update) a personalized threshold value for identifying the number of times the designated motions were performed, based on the user selecting the suggested value.

For example, the processor 410 may perform an algorithm for evaluating the user's exercise. The processor 410 may evaluate a posture for each of the designated motions. For example, the processor 410 may identify whether a designated motion (e.g., a squat motion) is performed with an incorrect posture and/or an incomplete motion.

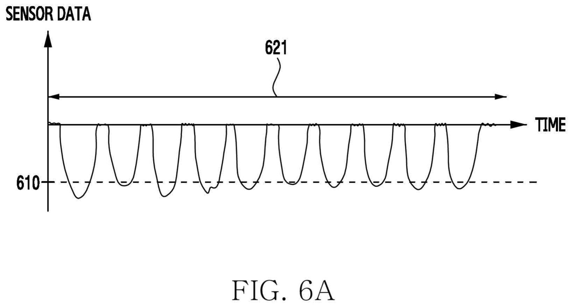

FIG. 6A is a graph illustrating an example operation of a wearable device for identifying the number of times designated motions were performed, according to various embodiments.

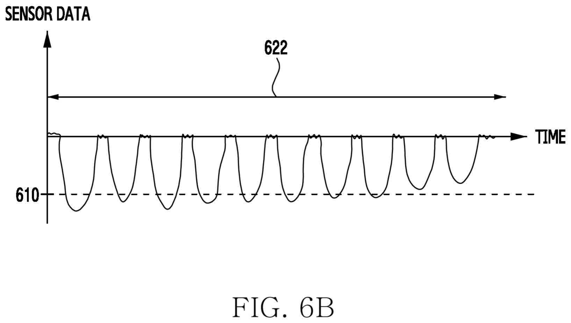

FIG. 6B is a graph illustrating an example operation of a wearable device for identifying the number of times designated motions were performed, according to various embodiments.

Referring to FIGS. 6A and 6B, the processor 410 of the wearable device 400 may identify whether a designated motion is performed using a first threshold value 610. For example, while one set formed of designated motions is being performed, the processor 410 of the wearable device 400 may obtain sensor data using the sensor 430. The processor 410 may identify a value of sensor data over time.

A value of sensor data described below may include an acceleration value in a designated direction (e.g., z-axis) of the wearable device 400, an angular velocity value in the designated direction of the wearable device 400, and an atmospheric pressure value identified by the wearable device 400. According to an embodiment, the value of the sensor data may be obtained, based on a combination (e.g., sum or average) of at least two values among an acceleration value in a designated direction (e.g., one of x-axis, y-axis, and z-axis), an angular velocity value in a designated direction (e.g., one of x-axis, y-axis, and z-axis), and an atmospheric pressure value identified by the wearable device 400. However, the disclosure is not limited thereto.

According to an embodiment, the processor 410 may identify the number of sections in which the value of the sensor data is less than or equal to the first threshold value 610. The processor 410 may identify the number of sections in which the value of the sensor data is less than or equal to the first threshold value 610 as the number of times designated motions were performed.

Referring to FIG. 6A, the processor 410 may identify the number of sections in which the value of the sensor data is less than or equal to the first threshold value 610 within the time section 621. The processor 410 may identify the number of sections in which the value of the sensor data is less than or equal to the first threshold value 610 among the time section 621 as 10. The processor 410 may identify that designated motions were performed 10 times within the time section 621.

For example, the processor 410 may identify the number of sections in which the value of the sensor data falls below the first threshold value 610 and rises above the first threshold value 610, within the time section 621. The processor 410 may identify the number of sections in which the value of the sensor data falls below the first threshold value 610 and rises above the first threshold value 610 among the time section 621 as 10. The processor 410 may identify that designated motions were performed 10 times within the time section 621.

Referring to FIG. 6B, the processor 410 may identify the number of sections in which the value of the sensor data is less than or equal to the first threshold value 610, within a time section 622. The processor 410 may identify the number of sections in which the value of the sensor data is less than or equal to the first threshold value 610 among the time section 622 as 8. The processor 410 may identify that designated motions were performed 8 times within the time section 622.

For example, the processor 410 may identify the number of sections in which the value of the sensor data falls below the first threshold value 610 and rises above the first threshold value 610 within the time section 622. The processor 410 may identify the number of sections in which the value of the sensor data falls below the first threshold value 610 and rises above the first threshold value 610 within the time section 622 as 8. The processor 410 may identify that designated motions were performed 8times within the time section 622.

Referring to FIGS. 6A and 6B, while a user's exercise based on designated motions is performed, the processor 410 may identify the number of times the designated motions were performed, based on a value of sensor data obtained using the sensor 430 (e.g., the acceleration sensor 431, the gyro sensor 432, the HR sensor 433, or the atmospheric pressure sensor 434). The processor 410 may provide the user with the number of times the designated motions were performed. The processor 410 may provide the user with the number of times the designated motions were performed, using the display 450.

For example, the first threshold value 610 may be fixed. For example, the first threshold value 610 may be set to a fixed value regardless of the user. Since an exercise performance ability, a posture, and/or a physical characteristic are different for each user, the number of times the designated motions were performed, which is identified using the first threshold value 610, may not be accurate according to the user. For example, when the user's posture is incorrect in a latter part of the exercise, the number of times the designated motions were performed, which is identified using the first threshold value 610, may be less than the number of times recognized by the user. Accordingly, the processor 410 may set a personalized threshold value so that the number of times the designated motions were performed, which is identified by the processor 410, corresponds to the number of times recognized by the user. Hereinafter, an example of an operation of the wearable device 400 (or the processor 410) for setting a second threshold value, which is a personalized threshold value, will be described in greater detail.

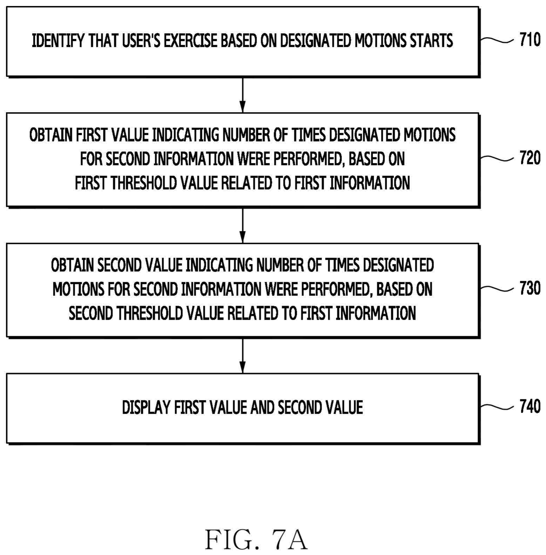

FIG. 7A is a flowchart illustrating an example operation of a wearable device according to various embodiments. In the following example, each operation may be performed sequentially, but is not necessarily performed sequentially. For example, the sequence of each operation may be changed, and at least two operations may be performed in parallel.

Referring to FIG. 7A, in operation 710, the processor 410 may identify that a user's exercise based on designated motions starts. For example, the user's exercise may include a squat exercise (e.g., the squat exercise 401 of FIG. 4A), a rowing machine exercise (e.g., the rowing machine exercise 402 of FIG. 4A), a dumbbell press exercise (e.g., the dumbbell press exercise 403 of FIG. 4A), and/or an arm curl exercise.

For example, the processor 410 may identify the number of sets in which the user's exercise is performed, the number of designated motions included in the set, and/or a break time between consecutive sets. The number of sets in which the user's exercise is performed, the number of designated motions included in the set, and/or the break time between consecutive sets may be set by the user, or be in a state of being stored in the memory 440.

For example, the memory 440 may store first information on exercise movement. The memory 440 may store first information including at least one threshold value (e.g., a first threshold value or a second threshold value) for identifying the number of times the designated motions were performed.

For example, the processor 410 may identify that the user's exercise based on the designated motions starts, based on a user input. For example, the processor 410 may identify a user input indicating a start of the exercise. The processor 410 may identify that the user's exercise starts based on the user input.

For example, the processor 410 may identify a user input for setting an exercise type (e.g., squat, rowing machine, dumbbell press, or arm curl), and identify that the user's exercise starts based on that a designated motion according to the set exercise type is performed.

According to an embodiment, the processor 410 may obtain second information on the user's exercise movement, based on identifying that the user's exercise starts. For example, the second information on the designated motions (or the user's exercise movement) may include sensor values (or sensor data) obtained through the sensor 430.

In operation 720, the processor 410 may obtain a first value indicating the number of times the designated motions for the second information were performed, based on a first threshold value related to the first information. For example, the processor 410 may obtain a first value indicating the number of times the designated motions were performed, using the first threshold value. For example, the processor 410 may use the first threshold value to obtain the first value indicating the number of times the designated motions were performed, based on the designated motions being performed.

For example, the first threshold value may be set based on at least one of the user's height, weight, body type, age, and/or gender. For example, the first threshold value may be a fixed value. For example, the first threshold value may be referred to as a default threshold value.

While one set is being performed, the processor 410 may obtain a first value indicating the number of times the designated motions were performed, using the first threshold value. The processor 410 may identify the number of time sections in which the value of the sensor data identified through the sensor 430 is less than or equal to the first threshold value as a first value. According to an embodiment, according to a type of value of sensor data and/or exercise type, the processor 410 may identify the number of time sections in which the value of the sensor data identified through the sensor 430 is greater than the first threshold value as a first value.

In operation 730, the processor 410 may obtain a second value indicating the number of times designated motions for the second information were performed, based on a second threshold value related to the first information.

According to an embodiment, the processor 410 may determine the second threshold value for identifying a value indicating the number of times the designated motions were performed as a second value distinct from the first value. For example, based on the first value corresponding to a designated value, the processor 410 may determine a second threshold value for identifying a value indicating the number of times the designated motions were performed as the second value distinct from the first value. The second threshold value may be related to the second information.

According to an embodiment, the processor 410 may identify that the first value corresponds to the designated value. For example, a plurality of sets may be set based on repetition according to a designated value of the designated motion. For example, the designated motion, which is repeated for the designated value, may form a set. The processor 410 may identify that a first set among the plurality of sets was performed, based on the first value corresponding to the designated value. As an example, the designated value may be changed based on a user input. As an example, the designated value may be changed, based on an exercise type and/or an exercise target (e.g., muscle hypertrophy, reducing fat, or increasing physical strength).

For example, the processor 410 may provide a notification indicating completion of the set to the user, based on the first value corresponding to the designated value. For example, the notification may be provided based on at least one of a screen change, a sound, and/or a vibration.

According to an embodiment, the processor 410 may determine a second threshold value for identifying a value indicating the number of times the designated motions were performed as the second value distinct from the first value. For example, in the case of identifying the number of times the designated motions were performed using the second threshold value distinct from the first threshold value, the processor 410 may identify a value indicating the number of times the designated motions were performed as the second value. The processor 410 may determine the second threshold value for identifying the value indicating the number of times the designated motions were performed as the second value. For example, the second threshold value may be a value that is changed by the user during exercise of the user. For example, the second threshold value may be referred to as a personalized threshold.

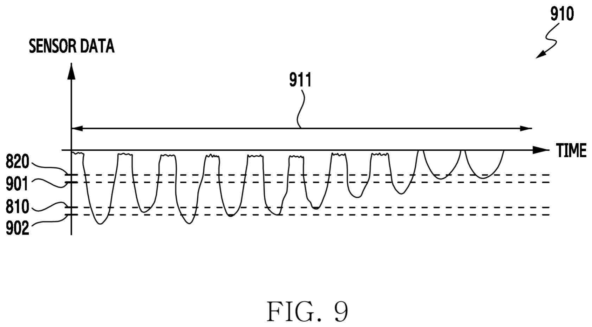

For example, the processor 410 may determine the second threshold value for identifying the value indicating the number of times the designated motions were performed as the second value, within a designated range. The designated range may be set based on a first reference value and a second reference value. The second reference value may be set to be larger than the first reference value. The processor 410 may determine the second threshold value within a designated range to prevent and/or reduce the occurrence of the second threshold value from being set too low or too high. When the second threshold value is determined within the designated range, the probability that the designated motions are incorrectly identified may be lower. For example, the processor 410 may determine the second threshold value as the first reference value, based on that the second threshold value for identifying the value indicating the number of times the designated motions were performed as the second value is identified to be less than the first reference value. As an example, the processor 410 may determine the second threshold value as the second reference value, based on that the second threshold value for identifying the value indicating the number of times the designated motions were performed as the second value is identified to be greater than the second reference value. A specific example in which the second threshold value is determined within a designated range will be described later in FIG. 9.

In operation 740, the processor 410 may display the first value and the second value. For example, the processor 410 may display the first value and the second value on the display 450. For example, the processor 410 may display a screen for an input for one of the first value and the second value. For example, based on determining the second threshold value, the processor 410 may display the screen for the input for one of the first value and the second value.

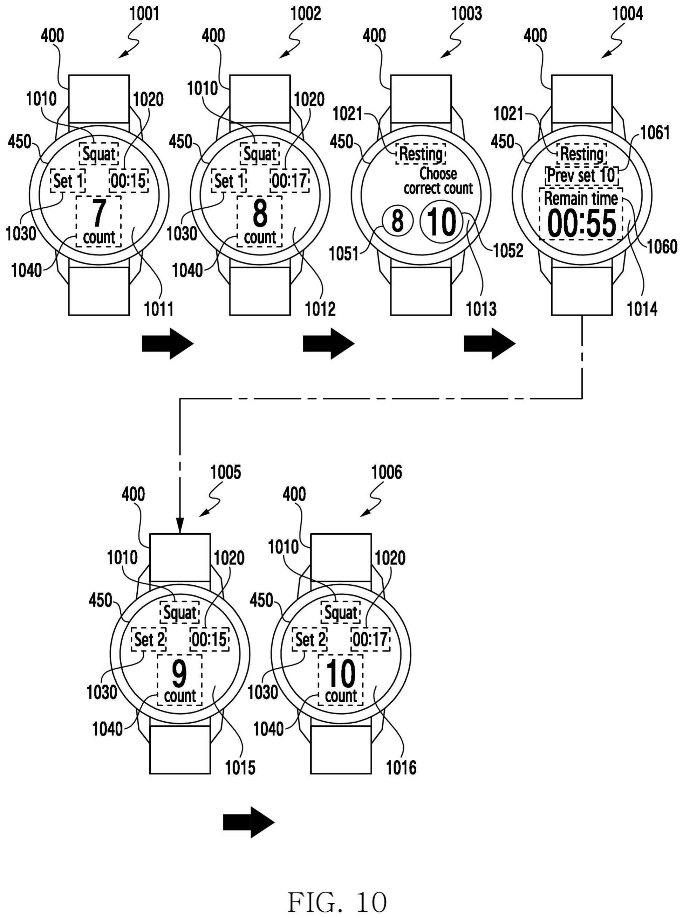

For example, based on determining the second threshold value, the processor 410 may display a screen including a first visual object representing the first value and a second visual object representing the second value. For example, the input for one of the first value and the second value may include an input for one of the first visual object and the second visual object. For example, the processor 410 may identify the input for one of the first value and the second value, based on the input for one of the first visual object and the second visual object. For example, a feature of the first visual object and a feature of the second visual object may be set differently. As an example, the second visual object may be highlighted compared to the first visual object in the screen. A size of the second visual object may be set to be larger than a size of the first visual object. As an example, a color of the first visual object may be set to be different from a color of the second visual object. For example, an example of the screen including the first visual object and the second visual object will be described in greater detail below with reference to FIG. 10.

According to an embodiment, based on identifying an input for the first value, the processor 410 may maintain a threshold value for identifying a value indicating the number of times the designated motions were performed (or the number of times the designated motions were performed) as the first threshold value. Based on identifying the input for the second value, the processor 410 may add the second threshold value to a threshold value for identifying a value indicating the number of times the designated motions were performed (or the number of times the designated motions were performed). The processor 410 may use both the first threshold value and the second threshold value to identify a value indicating the number of times the designated motions were performed (or the number of times the designated motions were performed). As an example, the processor 410 may identify the value indicating the number of times the designated motions were performed as a third value using the second threshold value, and identify the value indicating the number of times the designated motions were performed as a fourth value using the first threshold value. The processor 410 may display the third value through the display 450. While the third value is displayed through the display 450, the processor 410 may store the fourth value in the memory 440.

According to an embodiment, based on identifying the input for the first value, the processor 410 may maintain a threshold value for identifying the value indicating the number of times the designated motions were performed as the first threshold value. Based on identifying the input for the second value, the processor 410 may change the threshold value for identifying the number of times the designated motions were performed to the second threshold value.

For example, the processor 410 may display a screen for the input for one of the first value and the second value, based on completion of a first set of the plurality of sets. Based on identifying the input for the first value, the processor 410 may identify the third value indicating the number of times the designated motions were performed using the first threshold value in a second set after the first set. Based on identifying the input for the second value, the processor 410 may identify the third value indicating the number of times designated motions in the second set of after the first set were performed, using the second threshold value. The processor 410 may display a screen representing the third value while designated motions in the second set were performed. The processor 410 may increase the third value whenever the designated motions in the second set were performed. While the designated motions in the second set were performed, the processor 410 may identify a fourth value indicating the number of times the designated motions in the second set were performed using the first threshold value. The processor 410 may store the fourth value in the memory 440.

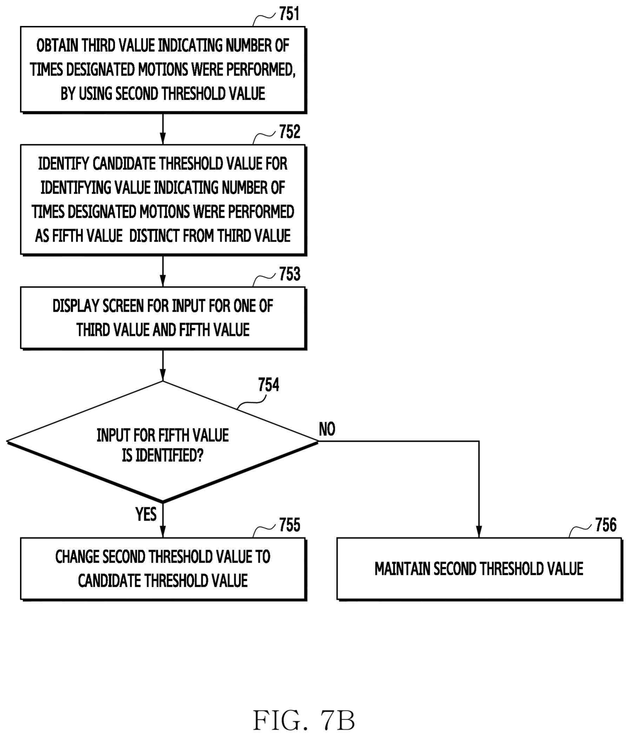

FIG. 7B is a flowchart illustrating an example operation of a wearable device according to various embodiments. In the following example, each operation may be performed sequentially, but is not necessarily performed sequentially. For example, the sequence of each operation may be changed, and at least two operations may be performed in parallel.

Referring to FIG. 7B, operations 751 to 756 may be performed after operation 740 of FIG. 7A is performed. For example, while the user performs a first set among a plurality of sets, the processor 410 of the wearable device 400 may perform operations 710 to 740 of FIG. 7A. While performing the second set, which is next set of the first set, the processor 410 may perform operations 751 to 756. However, the disclosure is not limited thereto. Operations 751 to 756 may be performed independently of the operations of FIG. 7A.

In operation 751, the processor 410 may obtain the third value indicating the number of times the designated motions were performed, using the second threshold value. For example, based on the designated motions being performed, the processor 410 may obtain the third value indicating the number of times the designated motions were performed, using the second threshold value.

While one set is being performed, the processor 410 may obtain the third value indicating the number of times the designated motions were performed, using the second threshold value. The processor 410 may identify the number of time sections in which a value of sensor data, which is identified through the sensor 430, is less than or equal to the second threshold value as a third value. According to an embodiment, the processor 410 may identify the number of time sections in which a value of the sensor data, which is identified through the sensor 430, is greater than the second threshold value as a third value, according to a type of sensor value and/or exercise type.

According to an embodiment, while the third value indicating the number of times the designated motions were performed is obtained using the second threshold value, the processor 410 may obtain a fourth value indicating the number of times the designated motions were performed using the first threshold value. For example, the processor 410 may not provide the fourth value to the user while the user performs the designated motions. The processor 410 may provide the third value to the user and store the fourth value in the memory 440. When displaying an exercise result after the exercise is completed, the processor 410 may provide the fourth value to the user. However, the disclosure is not limited thereto. According to an embodiment, the processor 410 may provide the third value and the fourth value to the user together. An operation of providing a value (e.g., the third value or the fourth value) to the user may refer, for example, to an operation of notifying the user of the value through various methods. For example, the processor 410 may provide the third value to the user by displaying the third value on the display 450. For example, the processor 410 may provide the third value to the user by outputting a sound (or voice) indicating the third value. For example, the processor 410 may provide the third value to the user by outputting a vibration indicating the third value.

In operation 752, the processor 410 may identify a candidate threshold value for identifying the value indicating the number of times the designated motions were performed as a fifth value distinct from the third value. For example, the candidate threshold value may be identified for determining whether to change the second threshold value.

For example, when identifying the number of times the designated motions were performed using the candidate threshold value, the processor 410 may identify the value indicating the number of times the designated motions were performed as the fifth value. The processor 410 may identify the candidate threshold value for identifying the value indicating the number of times the designated motions were performed as the fifth value.

For example, the processor 410 may identify the candidate threshold value for identifying the value indicating the number of times the designated motions were performed as the fifth value, within a designated range. The designated range may be set based on the first reference value and the second reference value. The processor 410 may set the candidate threshold value within the designated range, like the second threshold value. For example, operation 752 may correspond to operation 730 of FIG. 7A.

In operation 753, the processor 410 may display a screen for an input for one of the third value and the fifth value. Operation 753 may correspond to operation 740 of FIG. 7A.

In operation 754, the processor 410 may identify whether an input for the fifth value is identified. For example, the processor 410 may identify whether the input for the fifth value is identified, based on displaying the screen for the input for one of the third value and the fifth value.

In operation 755, when the input for the fifth value is identified, the processor 410 may change the second threshold value to the candidate threshold value. The processor 410 may change the second threshold value to the candidate threshold value based on the input for the fifth value. The processor 410 may identify that the user recognizes the number of times the designated motions were performed as the fifth value, based on the input to the fifth value. The processor 410 may change the second threshold value to the candidate threshold value, and perform operation 751 for identifying the number of times designated motions of the next set were performed using the second threshold value changed to the candidate threshold value.

In operation 756, when the input for the fifth value is not identified, the processor 410 may maintain the second threshold value. The processor 410 may maintain the second threshold value, based on that the input for the fifth value is not identified. For example, the processor 410 may maintain the second threshold value based on that the input for the third value is identified. The processor 410 may identify that the user recognizes the number of times the designated motions were performed as the third value, based on the input for the third value. The processor 410 may maintain the second threshold value, and perform operation 751 for identifying designated motions of the next set using the second threshold value.

According to an embodiment, the processor 410 may perform operations 751 to 756 whenever the user performs one set. For example, the processor 410 may repeatedly perform operations 751 to 756 until the user performs all of the plurality of sets. For example, the processor 410 may repeatedly perform operations 751 to 756 until the user terminates the exercise.

According to an embodiment, when the second threshold value is maintained based on termination of consecutive sets, operations 751 to 756 may not be performed.

According to an embodiment, the processor 410 may provide a result related to the user's exercise, based on completion of the user's exercise. For example, the processor 410 may display a screen for representing the result of the user's exercise through the display 450. For example, the screen for representing a result may represent a level for each of the designated motions.

For example, the second threshold value may be determined to be greater than the first threshold value. Based on that a value of the sensor data related to a first designated motion among designated motions is less than the first threshold value, the processor 410 may identify a level of the first designated motion as a first level (e.g., excellent). Based on that the value of the sensor data related to the first designated motion is less than the second threshold value and less than or equal to the first threshold value, the processor 410 may identify the level of the first designated motion as a second level (e.g., good). Based on the value of the sensor data related to the first designated motion is greater than or equal to the second threshold value, the processor 410 may identify the level of the first designated motion as a third level (e.g., bad).

According to an embodiment, the second threshold value may be determined to be smaller than the first threshold value according to a type of sensor data value and/or an exercise type. Based on that the value of the sensor data related to the first designated motion among the designated motions is greater than the first threshold value, the processor 410 may identify the level of the first designated motion as the first level (e.g., excellent). Based on that the value of the sensor data related to the first designated motion is less than or equal to the first threshold value and greater than the second threshold value, the processor 410 may identify the level of the first designated motion as the second level (e.g., good). Based on that the value of the sensor data related to the first designated motion is less than or equal to the second threshold value, the processor 410 may identify the level of the first designated motion as the third level (e.g., bad).

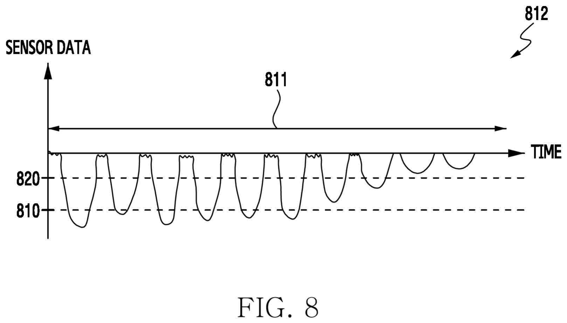

FIG. 8 is a graph illustrating an example operation of a wearable device according to various embodiments.

Referring to FIG. 8, a graph 812 illustrates an example of a change in a value of sensor data over time within a time section 811. For example, a user may perform designated motions (e.g., squat motions) within the time section 811. The processor 410 may obtain a first value indicating the number of times the designated motions were performed, using a first threshold value 810, based on that the user performs the designated motions within the time section 811. For example, the processor 410 may obtain a first value indicating the number of sections in which the value of the sensor data is less than or equal to the first threshold value 810. For example, the processor 410 may identify the first value indicating the number of sections in which the value of the sensor data is less than or equal to the first threshold value 810 as 6.

For example, the processor 410 may identify that the first value corresponds to a designated value (e.g., 6). The processor 410 may identify that one set has been completed, based on the first value corresponding to the designated value. After one set has been completed, the processor 410 may determine a second threshold value 820 for identifying a value indicating the number of times the designated motions were performed within the time section 811 as a second value distinct from the first value. For example, the processor 410 may identify the second value indicating the number of sections in which the value of the sensor data is less than or equal to the second threshold value 820. As an example, the processor 410 may identify the second value indicating the number of sections in which the value of the sensor data is less than or equal to the second threshold value 820 as 8.

For example, even when the user has performed eight designated motions during the time section 811, two of the eight designated motions may not be counted by the wearable device 400. Since a value of the sensor data related to the two designated motions is greater than the first threshold value 810 and less than or equal to the second threshold value 820, the processor 410 may identify the first value as 6. Therefore, a difference may be generated between the number of times the designated motions were performed, which is recognized by a user, and the number of times the designated motions were performed, which is identified by the processor 410. The processor 410 may determine the second threshold value 820 to further count the two designated motions. For example, the processor 410 may further count two designated motions, based on a pattern of the sensor data related to the designated motion. The processor 410 may determine the second threshold value 820 for further counting two designated motions. Based on determining the second threshold value 820, the processor 410 may display a screen for an input for one of a first value (e.g., 6) and a second value (e.g., 8). In a break time between consecutive sets, the processor 410 may determine the second threshold value 820, and display a screen for an input for one of the first value (e.g., 6) and the second value (e.g., 8).

According to an embodiment, after the second threshold value 820 is determined, when a change in the sensor data value over time in another set is identified such as graph 812, the processor 410 may identify the value indicating the number of times the designated motions were performed as 8. According to an embodiment, the processor 410 may identify a candidate threshold value for the second threshold value 820 whenever a plurality of sets are performed. The processor 410 may change (or update) or maintain the second threshold value 820, based on performing operations 751 to 756 of FIG. 7B whenever the user performs a set formed of designated motions. For example, when the second threshold value 820 is set too high in the first set, the second threshold value 820 may be changed based on a user input after the second set. When the user recognizes that the designated motion has not been properly performed, the processor 410 may identify a user input for setting the second threshold value 820 to be low through a screen after the second set. The processor 410 may change (or update) the second threshold value 820, based on receiving a user input whenever the user completes the set.

According to an embodiment, the processor 410 may identify second values distinguished from the first value. The processor 410 may determine second threshold values for identifying each of the second values. The processor 410 may display a screen for an input for one of the first value and the second value.

FIG. 9 is a graph illustrating an example operation of a wearable device according to various embodiments.

Referring to FIG. 9, a graph 910 illustrates an example of a change in a value of sensor data over time, within a time section 911. For example, a user may perform designated motions (e.g., squat operations) within the time section 911. The processor 410 may obtain a first value indicating the number of times the designated motions were performed, using the first threshold value 810, based on the user performing the designated motions within the time section 911. The processor 410 may identify a first value indicating the number of sections in which a value of the sensor data is less than or equal to the first threshold value 810 as 6.

For example, the processor 410 may identify that the first value corresponds to a designated value (e.g., 6). The processor 410 may identify that one set has been completed, based on the first value corresponding to the designated value. After one set has been completed, the processor 410 may determine a second threshold value 820 for identifying a value indicating the number of times the designated motions were performed within the time section 911 as a second value distinct from the first value.

According to an embodiment, the second threshold value 820 may be determined within a designated range. The processor 410 may determine the second threshold value 820 within a designated range. For example, the designated range may be set based on a reference value 901 and a reference value 902. The reference value 901 may be set to be greater than the reference value 902.

For example, the processor 410 may determine the second threshold value 820 as the reference value 901, based on identifying that the second threshold value 820 is greater than the reference value 901. Although not illustrated, the processor 410 may determine the second threshold value 820 as the reference value 902, based on identifying that the second threshold value 820 is less than the reference value 901.

Referring to FIG. 9, in case that the second threshold value 820 is set to be less than the reference value 902, even when the user performs the designated motion, the processor 410 may not identify that the designated motion was performed. In case that the second threshold value 820 is set to be greater than the reference value 901, even when the user has not performed the designated motion, the processor 410 may identify that the designated motion was performed. For example, the sensor data may be obtained using the atmospheric pressure 434. In case that hen the second threshold value 820 is set to be out of the designated range, the processor 410 may identify that the designated motion is performed even when an interval of vertical movement from the ground is short. For example, the sensor data may be obtained using the gyro sensor 432. When the second threshold value 820 is set to be out of the designated range, the processor 410 may identify that the designated motion is performed even in a case of highly irregular movements of a posture of the wearable device 400.

As described above, when the second threshold value 820 is out of a designated range set based on the reference value 901 and the reference value 902, a malfunction may occur. Accordingly, the processor 410 may determine the second threshold value 820 within the designated range.

FIG. 10 is a diagram illustrating an example of a screen of a wearable device according to various embodiments.

Referring to FIG. 10, a state of the wearable device 400 may be changed from a state 1001 to a state 1006.

In the state 1001 and a state 1002, while the user performs a first set, the processor 410 may display screens 1011 and 1012 related to the first set through the display 450 of the wearable device 400. The screens 1011 and 1012 of the states 1001 and 1002 may include a text 1010 indicating of an exercise type, a text 1020 indicating elapsed time of a set, a text 1030 indicating a set number, and a text 1040 indicating the number of times designated motions are identified while the user performs the first set.

According to an embodiment, while the user performs the first set, the processor 410 may identify a first value indicating the number of times the designated motions were performed using a first threshold value. In the state 1001, the processor 410 may identify the first value indicating the number of times the designated motions were performed as 7, using the first threshold value. The processor 410 may represent that the number of times the designated motions were performed is 7, through the text 1040. The processor 410 may represent that the number of times the designated motions were performed is 7, through the text 1040, within the screen 1011.

In the state 1002, the processor 410 may identify the first value indicating the number of times the designated motions were performed as 8, using the first threshold value. Based on identifying that the designated motion is performed one more time after the state 1001, the processor 410 may identify the first value indicating the number of times the designated motions were performed as 8. The processor 410 may indicate that the number of times the designated motions were performed is 8, based on changing the text 1040. Based on changing the text 1040, the processor 410 may indicate that the number of times the designated motions were performed is 8, within the screen 1012.

According to an embodiment, the processor 410 may identify that the first value (e.g., 8) corresponds to a designated value (e.g., 8). The processor 410 may change a state of the wearable device 400 from the state 1002 to the state 1003, based on identifying that the first value corresponds to the designated value. The processor 410 may identify that the first set has been completed, based on identifying that the first value corresponds to the designated value. The processor 410 may change the state of the wearable device 400 from the state 1002 to the state 1003, based on identifying that the first set has been completed.

In the state 1003, at a break time between the first set and the second set, the processor 410 may display a screen 1013 for an input for one of the first value and the second value through the display 450. For example, the processor 410 may display, on the screen 1013, a text 1021 indicating that it is a break time during exercise.

The processor 410 may display the screen 1013 for an input for one of the first value and the second value, based on displaying a first visual object 1051 and a second visual object 1052. For example, the processor 410 may determine a second threshold value for identifying a value indicating the number of times designated motions were performed in the first set as a second value (e.g., 10) distinct from the first value (e.g., 8). The processor 410 may display the first visual object 1051 indicating the first value on the screen 1013. The processor 410 may display the second visual object 1052 indicating the second value on the screen 1013.

For example, the second visual object 1052 may be highlighted in the screen 1013 compared to the first visual object 1051. As an example, a size of the second visual object 1052 may be set to be larger than a size of the first visual object 1051.

FIG. 10 illustrates an example in which only the first visual object 1051 and the second visual object 1052 are displayed, but the disclosure not limited thereto. The processor 410 may display second visual objects representing each of the second values (e.g., 7, 9, 10, and 11) on the screen.

According to an embodiment, the processor 410 may identify that the user has performed the designated motions 10 times in the first set, based on identifying an input for the second visual object 1052. The processor 410 may identify the number of times the designated motions were performed, using the second threshold value in the second set. The processor 410 may change a state of the wearable device 400 from the state 1003 to the state 1004, based on identifying an input for the second visual object 1052. According to an embodiment, the processor 410 may identify that the user has performed the designated motions eight times in the first set, based on identifying an input for the first visual object 1051. The processor 410 may identify the number of times the designated motions were performed using the first threshold value even in the second set.

In the state 1004, the processor 410 may display the screen 1014 based on an input for the second visual object 1052. The screen 1014 may include a text 1021 indicating a break time, a text 1061 indicating the number of times the designated motions were performed in the previous set, and/or a text 1060 indicating a remaining time for the break time. For example, since the input for the second visual object 1052 is performed, the processor 410 may display the number of times the designated motions were performed in the first set as 10.

According to an embodiment, the processor 410 may change the state of the wearable device 400 from the state 1004 to the state 1005, after all remaining time for the break time has elapsed.

In the state 1005 and the state 1006, the second set may start after all remaining time for the break time has elapsed. While the user performs the second set, the processor 410 may display screens 1015 and 1016 related to the second set through the display 450 of the wearable device 400. The screens 1015 and 1016 of the states 1005 and 1006 may include a text 1010 indicating of an exercise type, a text 1020 indicating elapsed time of a set, a text 1030 indicating a set number, and a text 1040 indicating the number of times designated motions are identified while the user performs the second set.

The processor 410 may identify a third value indicating the number of times the designated motions were performed, using the second threshold value, while the user performs the second set.

In the state 1005, the processor 410 may identify the third value indicating the number of times the designated motions were performed as 9, using the second threshold value. The processor 410 may indicate that the number of times the designated motions were performed is 9, through the text 1040. The processor 410 may indicate that the number of times the designated motions were performed is 9 through the text 1040, within the screen 1015.

In the state 1006, the processor 410 may identify the third value indicating the number of times the designated motions were performed as 10, using the second threshold value. Based on identifying that the designated motions are performed one more time after the state 1005, the processor 410 may identify the third value indicating the number of times the designated motions were performed as 10. The processor 410 may indicate that the number of times the designated motions were performed is 10, based on changing the text 1040. Based on changing the text 1040, the processor 410 may indicate that the number of times the designated motions were performed is 10, within the screen 1016. According to an embodiment, the processor 410 may obtain a fourth value indicating the number of times the designated motions were performed using the first threshold value while the second set is in progress. The processor 410 may store the fourth value in the memory 440. According to an embodiment, after the second set is terminated, the processor 410 may obtain the fourth value indicating the number of times the designated motions were performed using the first threshold value. The processor 410 may store the fourth value in the memory 440.

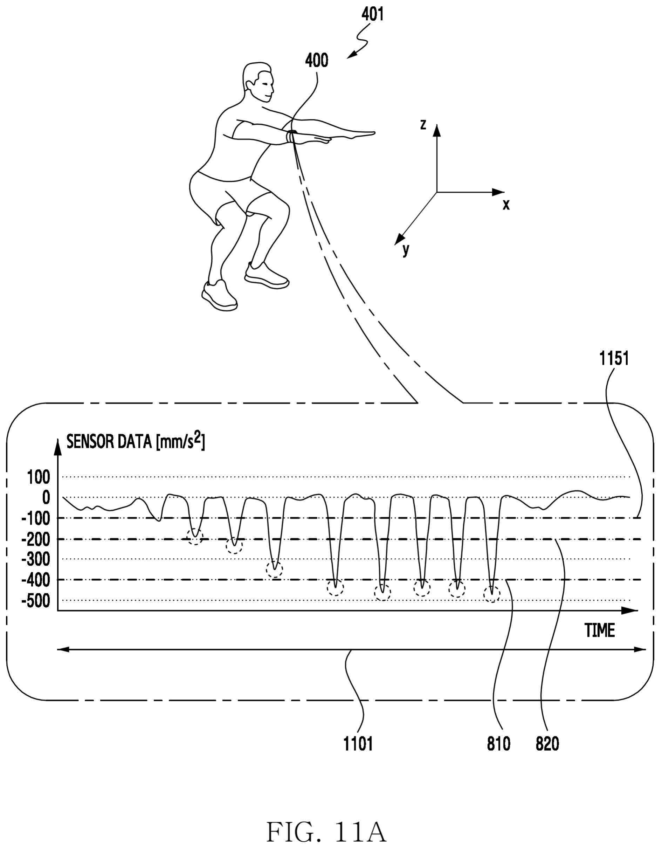

FIG. 11A is a diagram including a graph illustrating an example operation of a wearable device for a squat exercise according to various embodiments.

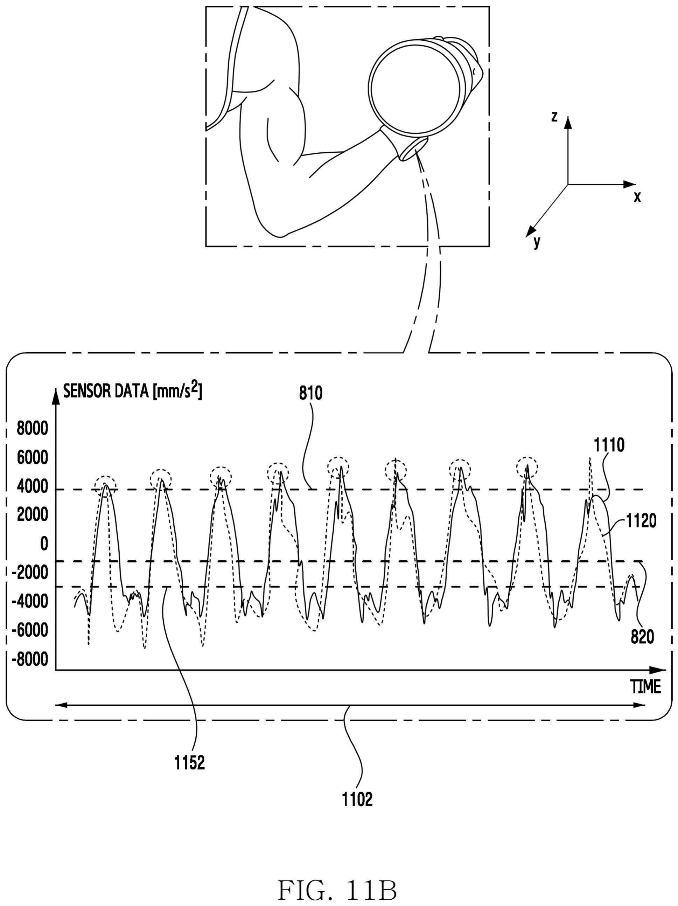

FIG. 11B is a diagram including a graph illustrating an example operation of a wearable device for an arm curl exercise according to an embodiment.

Referring to FIGS. 11A and 11B, sensor data for setting a first threshold value and a second threshold value may be variously set. In the above-described embodiment, although an example in which sensor data is configured with one value has been described for convenience of explanation, the sensor data may be complex data obtained using at least one of an acceleration sensor 431, a gyro sensor 432, and/or an atmospheric pressure sensor 434. For example, while a squat exercise is being performed, the processor 410 may identify a squat motion using sensor data related to vertical movement in a z-axis direction (e.g., the ground direction) of the acceleration sensor 431. For example, while the squat exercise is being performed, the processor 410 may identify the squat motion, using both sensor data obtained using the acceleration sensor 431 and sensor data obtained using the gyro sensor 432. The processor 410 may determine a first designated value for identifying the squat motion based on sensor data obtained using the acceleration sensor 431 and a second designated value for identifying the squat motion based on sensor data obtained using the gyro sensor 432. A threshold value for identifying the squat motion may include the first designated value and the second designated value.

In FIG. 11A, while the user performs a squat exercise in a time section 1101, the processor 410 may identify the user's squat motion based on sensor data including an acceleration value in the z-axis direction (or the ground direction) obtained using the acceleration sensor 431. A unit of the acceleration value may be set to [mm/s2].

For example, the processor 410 may identify the number of time sections in which the acceleration value is less than or equal to a first threshold value 810 based on the sensor data. The processor 410 may identify the number of time sections in which the acceleration value is less than or equal to the first threshold value 810 as 5. The processor 410 may identify that the number of times squat operations were performed is 5, using the first threshold value 810.

For example, the processor 410 may identify the number of time sections in which the acceleration value is less than or equal to the second threshold value 820, based on the sensor data. The processor 410 may identify the number of time sections in which the acceleration value is less than or equal to the second threshold value 820 as 7. The processor 410 may identify that the number of times squat operations were performed is 7, using the second threshold value 820.

For example, the processor 410 may identify the number of time sections in which the acceleration value is less than or equal to a reference value 1151, based on the sensor data. The processor 410 may identify the number of time sections in which the acceleration value is less than or equal to the reference value 1151 as 8. The processor 410 may identify that the number of times squat operations were performed is 8, using the reference value 1151. For example, when the second threshold value is increased to the reference value 1151, the processor 410 may use the second threshold value to identify that the number of times squat operations were performed is 8.

In FIG. 11B, while the user performs an arm curl exercise in a time section 1102, the processor 410 may obtain sensor data including an acceleration value in the z-axis direction (or the ground direction) and an acceleration value in the x-axis direction, which are obtained using the acceleration sensor 431. The processor 410 may identify an arm curl motion of the user using the sensor data. The unit of the acceleration value may be set to [mm/s2]. Since the arm curl motion has a semi-circular exercise radius, a change in acceleration in the z-axis direction and the x-axis direction may occur.

A graph 1110 represents an acceleration value in the z-axis direction over time. A graph 1120 represents an acceleration value in the x-axis direction over time. Based on the arm curl motion of the user, the acceleration value in the z-axis direction and the acceleration value in the x-axis direction may be changed. The processor 410 may set the first threshold value 810 and the second threshold value 820 based on the acceleration value in the z-axis direction and the acceleration value in the x-axis direction.

For example, the processor 410 may identify the number of time sections in which both the acceleration value in the z-axis direction and the acceleration value in the x-axis direction are greater than or equal to the first threshold value 810. The processor 410 may identify the number of time sections in which both the acceleration value in the z-axis direction and the acceleration value in the x-axis direction are greater than or equal to the first threshold value 810 as 8. The processor 410 may identify that the number of times the arm curl motion is performed is 8, using the first threshold value 810.

For example, the processor 410 may identify the number of time sections in which both the acceleration value in the z-axis direction and the acceleration value in the x-axis direction are greater than or equal to the second threshold value 820. The processor 410 may identify the number of time sections in which both the acceleration value in the z-axis direction and the acceleration value in the x-axis direction are greater than or equal to the second threshold value 820 as 9. The processor 410 may identify that the number of times the arm curl motion is performed is 9, using the second threshold value 820. For example, in a case that the second threshold value 820 is used, the processor 410 may further count the user's last two arm curl motions compared to a case that the first threshold value 810 is used.

For example, the processor 410 may identify the number of time sections in which both the acceleration value in the z-axis direction and the acceleration value in the x-axis direction are greater than or equal to a reference value 1152. The processor 410 may identify the number of time sections in which both the acceleration value in the z-axis direction and the acceleration value in the x-axis direction are greater than or equal to the reference value 1152 as 10. The processor 410 may identify that the number of times the arm curl motion is performed is 10, using the reference value 1152. For example, in a case that the reference value 1152 is used, the processor 410 may further count the user's last arm curl motion compared to a case that the second threshold value 820 is used. For example, when the second threshold value 820 is reduced to the reference value 1152, the processor 410 may use the second threshold value 820 to identify that the number of times the arm curl motions were performed is 10.

Referring to FIGS. 11A and 11B, as the second threshold value 820 is changed, the number of times designated motions are identified may be changed. For example, the processor 410 may use the first threshold value 810 to identify the number of times designated motions have been performed, and use the second threshold value 820 being changed according to the user input to identify the number of times designated motions have been performed. The processor 410 may identify the number of times the designated motions were performed, using the second threshold value 820, which is a personalized threshold value according to a user.

The reference value 1151 and the reference value 1152 may be set to prevent and/or reduce the designated motion from being identified even when the user does not perform the designated motion. For example, the reference value 1151 (or the reference value 1152) may be set based on the first threshold value 810. As an example, the reference value 1151 (or the reference value 1152) may be set to 50% of the first threshold value 810.

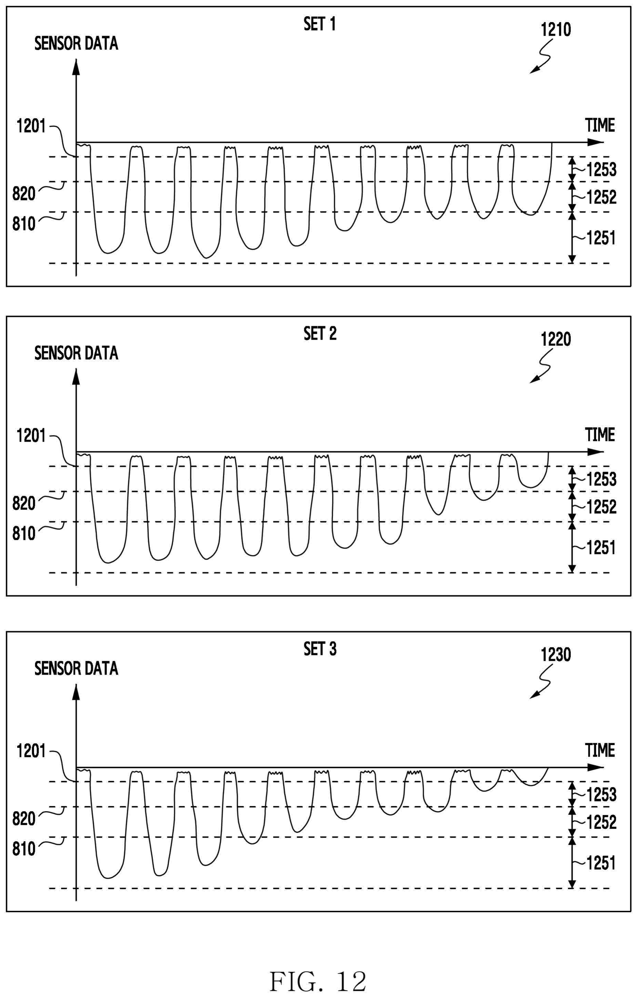

FIG. 12 includes graphs illustrating example operations of a wearable device according to various embodiments.

Referring to FIG. 12, when the second threshold value 820 is changed based on a user input, the processor 410 may also count a designated motion performed in a wrong posture, which is capable of affecting negatively the exercise effect according to a user's judgment. Accordingly, the processor 410 may evaluate each of designated motions after the user's exercise is terminated. For example, the processor 410 may identify one of a plurality of levels for each of the designated motions. For example, the processor 410 may identify each of designated motion as one of a first level (e.g., excellent), a second level (e.g., good), and a third level (e.g., bad).

A graph 1210 represents a change in a value (e.g., an acceleration value in the ground direction) of sensor data according to a time identified in a first set. A graph 1220 represents a change in a value (e.g., an acceleration value in the ground direction) of sensor data according to a time identified in a second set. A graph 1230 represents a change in a value (e.g., an acceleration value in the ground direction) of sensor data according to a time identified in a third set.

According to an embodiment, after the user's exercise of the first, second, and third sets is completed, the processor 410 may identify a level of each of designated motions using the first threshold value 810 and the second threshold value 820 at the time when the exercise is terminated. For example, the processor 410 may identify a level of a first designated motion as the first level (e.g., excellent) based on that a value of sensor data related to the first designated motion among the designated motions is less than the first threshold value 810. The processor 410 may identify the level of the first designated motion as the second level (e.g., good), based on that the value of the sensor data related to the first designated motion among the designated motions is greater than or equal to the first threshold value 810 and less than the second threshold value 820. The processor 410 may identify the level of the first designated motion as the third level (e.g., bad), based on that the value of the sensor data related to the first designated motion among the designated motions is greater than or equal to the second threshold value 820 and less than the reference value 1201.

The processor 410 may identify three sections, based on the first threshold value 810, the second threshold value 820, and the reference value 1201. The processor 410 may identify a section in which the value of the sensor data is less than the first threshold value 810 as a first section 1251. The processor 410 may identify a section in which the value of the sensor data is greater than or equal to the first threshold value 810 and less than the second threshold value 820 as a second section 1252. The processor 410 may identify a section in which the value of the sensor data is greater than or equal to the second threshold value 820 and less than the reference value 1201 as a third section 1253.

Referring to the graph 1210, the number of time sections in which the value of the sensor data is within the first section 1251 may be identified as 10. There may be no time section in which the value of the sensor data is within the second section 1252 and the time section in which the value of the sensor data is within the third section 1253. The processor 410 may identify that a level of 10 designated motions performed in a first set is the first level.

Referring to the graph 1220, the number of time sections in which the value of the sensor data is within the first section 1251 may be identified as 7. The number of time sections in which the value of the sensor data is within the second section 1252 may be identified as 2. The number of time sections in which the value of the sensor data is within the third section 1253 may be identified as 1. The processor 410 may identify that, among the 10 designated motions performed in the second set, a level of 7 designated motions is the first level, a level of 2 designated motions is the second level, and a level of 1 designated motion is the third level.

Referring to the graph 1230, the number of time sections in which the value of the sensor data is within the first section 1251 may be identified as 4. The number of time sections in which the value of the sensor data is within the second section 1252 may be identified as 4. The number of time sections in which the value of the sensor data is within the third section 1253 may be identified as 2. The processor 410 may identify that, among the 10 designated motions performed in the third set, a level of 4 designated motions is the first level, a level of 4 designated motions is the second level, and a level of 2 designated motions is the third level.

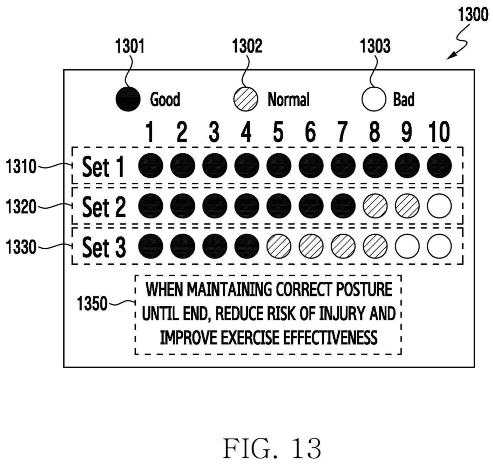

According to an embodiment, after the exercise is completed, the processor 410 may display a screen for providing an exercise result on the display 450. A specific example of the screen for providing the exercise result will be described in greater detail below with reference to FIG. 13.

FIG. 13 is a diagram illustrating an example operation of a wearable device for displaying a result of an exercise of a user, according to various embodiments.

Referring to FIG. 13, the processor 410 may display a screen 1300 for providing an exercise result on the display 450 after the exercise is completed. For example, the processor 410 may display a level for each of designated motions as one of an object 1301, an object 1302, and an object 1303. For example, the object 1301 may represent a first level. The object 1302 may represent a second level. The object 1303 may represent a third level.

Levels for each of 10 designated motions of a first set (e.g., set 1) may be displayed in an area 1310. For example, levels of 10 designated motions of the first set may all be identified as the first level.

Levels for each of 10 designated motions of a second set (e.g., set 2) may be displayed in an area 1320. For example, among the 10 designated motions of the second set, levels of 7 designated motions may be identified as the first level. Among the 10 designated motions of the second set, levels of 2 designated motions may be identified as the second level. Among the 10 designated motions of the second set, a level of 1 designated motion may be identified as the third level.

Levels for each of 10 designated motions of a third set (e.g., set 3) may be displayed in an area 1330. For example, among the 10 designated motions of the third set, levels of 4 designated motions may be identified as the first level. Among the 10 designated motions of the second set, levels of 4 designated motions may be identified as the second level. Among the 10 designated motions of the second set, levels of 2 designated motions may be identified as the third level.

According to an embodiment, the screen 1300 may further include an area 1350 for displaying text indicating feedback on the exercise result. Although not illustrated, the processor 410 may display text indicating that last 9 and 10 postures of the third set are unstable and suggesting that a correct posture should be maintained until the end, in the area 1350. Although not illustrated, when results for exercise using a dumbbell (or a barbell) are displayed, the processor 410 may display text guiding the user to reduce the weight of the dumbbell (or barbell) in a set in which an exercise posture is unstable.

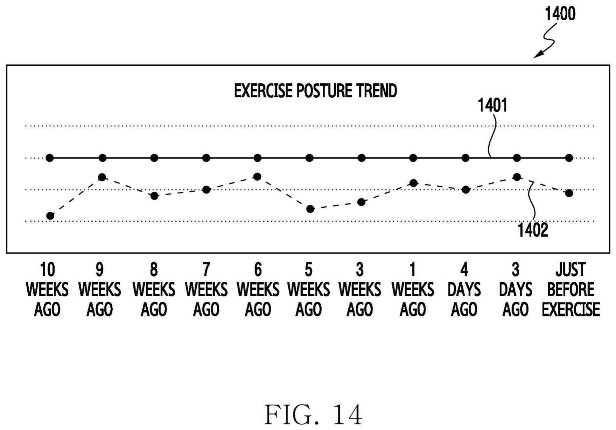

FIG. 14 is a graph illustrating an example operation of a wearable device for displaying a result of an exercise of a user, according to various embodiments.

Referring to FIG. 14, the processor 410 may provide a screen 1400 indicating a change in the first threshold value 810 and the second threshold value 820 to the user. For example, the first threshold value 810 may be a fixed value and may not change. The second threshold value 820 may be changed according to a user input. Accordingly, the second threshold value 820 may be changed whenever the user performs exercise.

The processor 410 may store results of a plurality of exercises in the memory 440. For example, based on the results of the plurality of exercises, the processor 410 may identify a trend in which the first threshold value 810 is changed and a trend in which the second threshold value 820 is changed and provide it through the screen 1400.

For example, the processor 410 may identify a trend in which the first threshold value 810 is changed and a trend in which the second threshold value 820 is changed according to 11 exercise results. A graph 1401 may represent a trend in which the first threshold value 810 is changed. A graph 1402 may represent a trend in which the second threshold value 820 is changed.

Although not illustrated, the processor 410 may display a ratio of the second threshold value 820 with respect to the first threshold value 810, at substantially the same time point (e.g., 9 weeks ago). For example, the processor 410 may display the ratio of the second threshold value 820 with respect to the first threshold value 810 as 80% based on the exercise result of 6weeks ago. Although not illustrated, the processor 410 may display a rate of change of the second threshold value 820 at different time points. The processor 410 may display the rate of change of the second threshold value 820 as 10%, based on the exercise result 7 weeks ago and the exercise result 6 weeks ago.

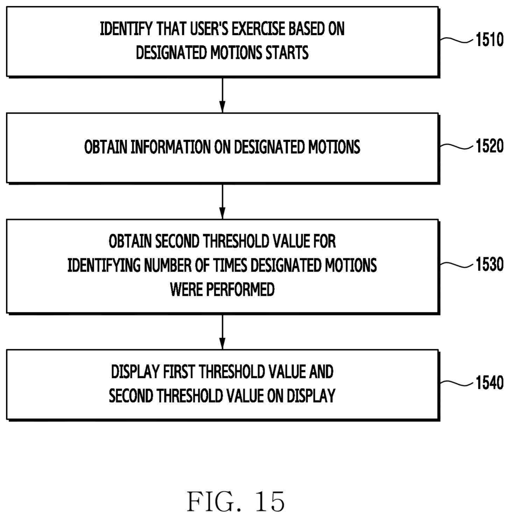

FIG. 15 is a flowchart illustrating an example operation of a wearable device according to various embodiments.

Referring to FIG. 15, in operation 1510, the processor 410 may identify that a user's exercise based on designated motions starts. For example, the user's exercise may include a squat exercise (e.g., the squat exercise 401 of FIG. 4A), a rowing machine exercise (e.g., the rowing machine exercise 402 of FIG. 4A), a dumbbell press exercise (e.g., the dumbbell press exercise 403 of FIG. 4A), and/or an arm curl exercise. For example, operation 1510 may correspond to operation 710 of FIG. 7A.

For example, the memory 440 may store a first threshold value. The memory 440 may store the first threshold value for identifying the number of times the designated motions were performed.

For example, the first threshold value may be set based on at least one of the user's height, weight, body type, age, and/or gender. For example, the first threshold value may be a fixed value. For example, the first threshold value may be referred to as a default threshold value.

In operation 1520, the processor 410 may obtain information on the designated motions. For example, the processor 410 may obtain information on the designated motions, based on identifying that the user's exercise starts. For example, information on the designated motions (or a user's exercise movement) may include sensor values (or sensor data) obtained through the sensor 430.

In operation 1530, the processor 410 may obtain a second threshold value for identifying the number of times the designated motions were performed. For example, based on the information on the designated motions, the processor 410 may obtain the second threshold value for identifying the number of times the designated motions were performed. For example, the processor 410 may obtain a second threshold value for identifying the number of times the designated motions were performed, which is distinguished from the first threshold value.

In operation 1540, the processor 410 may display the first threshold value and the second threshold value on the display 450. For example, the first threshold value and the second threshold value may be used to identify the number of times the designated motions were performed.

According to an embodiment, the processor 410 may display a screen for an input for one of the first threshold value and the second threshold value on the display 450. For example, the processor 410 may identify each of the first threshold value and the second threshold value as one of 'high', 'middle', and 'low'. The processor 410 may display a screen for an input for one of the first threshold value set to 'middle' and the second threshold value set to 'low', on the display 450.

According to an embodiment, a screen for the input for one of the first threshold value and the second threshold value may include a first visual object related to the first threshold value and a second visual object related to the second threshold value. The input for one of the first threshold value and the second threshold value may include an input for one of the first visual object and the second visual object. For example, the second visual object may be highlighted on the screen, compared to the first visual object.

According to an embodiment, the processor 410 may maintain a threshold value for identifying the number of times the designated motions were performed as the first threshold value, based on identifying an input for the first threshold value. The processor 410 may change the threshold value for identifying the number of times the designated motions were performed from the first threshold value to the second threshold value, based on identifying an input for the second threshold value.

According to example embodiment, a wearable device may comprise a display, at least one sensor, a first memory storing first information on exercise movement, comprising one or more storage media, a second memory storing instructions, comprising one or more storage media, a at least one processor comprising processing circuitry. The instructions, when executed by the at least one processor individually or collectively, cause the wearable device to identify that an exercise of a user based on designated motions is started. The instructions, when executed by the at least one processor individually or collectively, cause the wearable device to obtain second information on exercise movement of the user, in accordance with performance of the designated motions. The instructions, when executed by the at least one processor individually or collectively, cause the wearable device to obtain, based on the second information, a first value indicating a number of times the designated motions were performed, using a first threshold value included in the first information. The instructions, when executed by the at least one processor individually or collectively, cause the wearable device to determine a second threshold value for identifying a value indicating the number of times the designated motions were performed, as a second value distinguished from the first value. The instructions, when executed by the at least one processor individually or collectively, cause the wearable device to display a screen for an input for one of the first value and the second value, based on determining the second threshold value.

According to example embodiment, the instructions, when executed by the at least one processor individually or collectively, cause the wearable device to display the screen including a first visual object representing the first value and a second visual object representing the second value, based on determining the second threshold value.

According to example embodiment, the input for one of the first value and the second value may include an input for one of the first visual object and the second visual object.

According to example embodiment, the second visual object may be highlighted compared to the first visual object in the screen.

According to example embodiment, The instructions, when executed by the at least one processor individually or collectively, cause the wearable device to, based on identifying an input for the first threshold value, maintain a threshold value for identifying a value indicating the number of times the designated motions were performed, as the first threshold value. The instructions, when executed by the at least one processor individually or collectively, cause the wearable device to, based on identifying an input for the second threshold value, change the threshold value for identifying the value indicating the number of times the designated motions were performed, from the first threshold value to the second threshold value.

According to example embodiment, The instructions, when executed by the at least one processor individually or collectively, cause the wearable device to, based on identifying an input for the first value, maintain a threshold value for identifying a value indicating the number of times the designated motions were performed, as the first threshold value. The instructions, when executed by the at least one processor individually or collectively, cause the wearable device to, based on identifying an input for the second value, add the second threshold value to the threshold value for identifying the value indicating the number of times the designated motions were performed.

According to example embodiment, The instructions, when executed by the at least one processor individually or collectively, cause the wearable device to determine the second threshold value for identifying the value indicating the number of times the designated motions were performed, as the second value, within a designated range.

According to example embodiment, the designated range may be set based on a first reference value and a second reference value greater than the first reference value. The instructions, when executed by the at least one processor individually or collectively, cause the wearable device to determine the second threshold value as the first reference value, based on identifying that the second threshold value is less than the first reference value. The instructions, when executed by the at least one processor individually or collectively, cause the wearable device to determine the second threshold value as the second reference value, based on identifying that the second threshold value is greater than the second reference value.

According to example embodiment, the second threshold value may be determined to be less than the first threshold value. The instructions, when executed by the at least one processor individually or collectively, cause the wearable device to, based on a value of sensor data related to a first designated motion among the designated motions being greater than or equal to the first threshold value, identify a level of the first designated motion as a first level. The instructions, when executed by the at least one processor individually or collectively, cause the wearable device to, based on the the value of the sensor data being less than the first threshold value and greater than or equal to the second threshold value, identify the level of the first designated motion as a second level. The instructions, when executed by the at least one processor individually or collectively, cause the wearable device to, based on that the value of the sensor data is less than the second threshold value, identify the level of the first designated motion as a third level.

According to example embodiment, the second threshold value may be determined to be greater than the first threshold value. The instructions, when executed by the at least one processor individually or collectively, cause the wearable device to, based on a value of sensor data related to a first designated motion among the designated motions being less than the first threshold value, identify a level of the first designated motion as a first level. The instructions, when executed by the at least one processor individually or collectively, cause the wearable device to, based on the value of the sensor data being greater than or equal to the first threshold value and less than the second threshold value, identify the level of the first designated motion as a second level. The instructions, when executed by the at least one processor individually or collectively, cause the wearable device to, based on the value of the sensor data being greater than or equal to the second threshold value, identify the level of the first designated motion as a third level.

According to example embodiment, The instructions, when executed by the at least one processor individually or collectively, cause the wearable device to display a screen for displaying results of the exercise of the user based on completion of the exercise of the user. The screen for displaying the results may represent a level related to each of the designated motions.

According to example embodiment, the exercise of the user may be performed through a plurality of sets set based on repetitions of the designated motion according to a designated value. The instructions, when executed by the at least one processor individually or collectively, cause the wearable device to identify, based on the first value corresponding to the designated value, that a first set among the plurality of sets are performed.

According to example embodiment, The instructions, when executed by the at least one processor individually or collectively, cause the wearable device to provide, based on the first value corresponding to the designated value, a notification representing end of the first set.

According to example embodiment, The instructions, when executed by the at least one processor individually or collectively, cause the wearable device to identify, based on an input for the second value, a third value indicating the number of times designated motions of a second set which is next set of the first set, among the plurality of sets, are performed, using the second threshold value.

According to example embodiment, The instructions, when executed by the at least one processor individually or collectively, cause the wearable device to display a screen representing the third value indicating the number of times the designated motions of the second set are performed using the second threshold value, based on the designated motions of the second set are being performed.

According to example embodiment, The instructions, when executed by the at least one processor individually or collectively, cause the wearable device to store a fourth value indicating the number of times the designated motions of the second set are performed using the first threshold value, while the designated motions of the second set are being performed.

According to example embodiment, a method performed by a wearable device may comprise obtaining second information on exercise movement of a user, in accordance with performance of the designated motions. The method may comprise obtaining, based on the second information, a first value indicating a number of times the designated motions were performed, using a first threshold value included in first information on exercise movement stored in a memory of the wearable device. The method may comprise determining a second threshold value for identifying a value indicating the number of times the designated motions were performed, as a second value distinguished from the first value. The method may comprise displaying a screen for an input for one of the first value and the second value, based on determining the second threshold value.

According to example embodiment, the method may comprise displaying the screen including a first visual object representing the first value and a second visual object representing the second value, based on determining the second threshold value. The input for one of the first value and the second value may include an input for one of the first visual object and the second visual object.

According to example embodiment, the second visual object may be highlighted compared to the first visual object in the screen.

According to example embodiment, the method may comprise, based on identifying an input for the first threshold value, maintaining a threshold value for identifying a value indicating the number of times the designated motions were performed, as the first threshold value. The method may comprise, based on identifying an input for the second threshold value, changing the threshold value for identifying the value indicating the number of times the designated motions were performed, from the first threshold value to the second threshold value.

According to example embodiment, a non-transitory computer-readable storage medium may store one or more programs. The one or more programs may comprise instructions which, when executed by at least one processor of a wearable device with a display, individually and/or collectively, cause the wearable device to identify that an exercise of a user based on designated motions is started. The one or more programs may comprise instructions which, when executed by the at least one processor, cause the wearable device to obtain second information on exercise movement of the user, in accordance with performance of the designated motions. The one or more programs may comprise instructions which, when executed by the at least one processor, cause the wearable device to obtain, based on the second information, a first value indicating a number of times the designated motions were performed, using a first threshold value included in the first information. The one or more programs may comprise instructions which, when executed by the at least one processor, cause the wearable device to determine a second threshold value for identifying a value indicating the number of times the designated motions were performed, as a second value distinguished from the first value. The one or more programs may comprise instructions which, when executed by the at least one processor, cause the wearable device to display a screen for an input for one of the first value and the second value, based on determining the second threshold value.

According to example embodiment, a wearable device may comprise a display, at least one sensor, a first memory storing first information on exercise movement, comprising one or more storage media, a second memory storing instructions, comprising one or more storage media, and at least one processor comprising processing circuitry. The instructions, when executed by the at least one processor individually or collectively, cause the wearable device to identify that an exercise of a user based on designated motions is started. The instructions, when executed by the at least one processor individually or collectively, cause the wearable device to obtain second information on the designated motions. The instructions, when executed by the at least one processor individually or collectively, cause the wearable device to obtain, based on a first threshold value related to the first information, a first value indicating the number of times the designated motions with respect to the second information were performed. The instructions, when executed by the at least one processor individually or collectively, cause the wearable device to obtain, based on a second threshold value related to the first information, a second value indicating the number of times the designated motions with respect to the second information were performed. The instructions, when executed by the at least one processor individually or collectively, cause the wearable device to display the first value and the second value on the display.

According to example embodiment, The instructions, when executed by the at least one processor individually or collectively, cause the wearable device to display a screen for an input for at least one of the first value and the second value. The screen may comprise a first visual object representing the first value and a second visual object representing the second value.

According to example embodiment, the input for at least one of the first value and/or the second value may comprise an input for at least one of the first visual object and/or the second visual object.

According to example embodiment, the second visual object may be highlighted compared to the first visual object in the screen.

According to example embodiment, The instructions, when executed by the at least one processor individually or collectively, cause the wearable device to, based on identifying an input for the first value, maintain a threshold value for identifying the number of times the designated motions were performed, included in the first information, as the first threshold value. The instructions, when executed by the at least one processor individually or collectively, cause the wearable device to, based on identifying an input for the second value, change the threshold value for identifying the number of times the designated motions were performed, included in the first information, from the first threshold value to the second threshold value.

According to example embodiment, The instructions, when executed by the at least one processor individually or collectively, cause the wearable device to, based on identifying an input for the first value, maintain a threshold value for identifying a number of times the designated motions were performed, comprised in the first information, as the first threshold value. The instructions, when executed by the at least one processor individually or collectively, cause the wearable device to, based on identifying an input for the second value, add the second threshold value to the threshold value for identifying the number of times the designated motions were performed, included in the first information.

According to example embodiment, The instructions, when executed by the at least one processor individually or collectively, cause the wearable device to determine the second threshold value for obtaining the second value, within a designated range.

According to example embodiment, the designated range may be set based on a first reference value and a second reference value which is greater than the first reference value. The instructions, when executed by the at least one processor individually or collectively, cause the wearable device to, based on identifying that the second threshold value is less than the first reference value, determine the second threshold value as the first reference value. The instructions, when executed by the at least one processor individually or collectively, cause the wearable device to, based on identifying that the second threshold value is greater than the second reference value, determine the second threshold value as the second reference value.

According to example embodiment, the second threshold value may be determined to be less than the first threshold value. The instructions, when executed by the at least one processor individually or collectively, cause the wearable device to, based on a value of sensor data related to a first designated motion among the designated motions being greater than or equal to the first threshold value, identify a level of the first designated motion as a first level. The instructions, when executed by the at least one processor individually or collectively, cause the wearable device to, based on the value of the sensor data being less than the first threshold value and greater than or equal to the second threshold value, identify the level of the first designated motion as a second level. The instructions, when executed by the at least one processor individually or collectively, cause the wearable device to, based on the value of the sensor data being less than the second threshold value, identify the level of the first designated motion as a third level.

According to example embodiment, the second threshold value may be determined to be greater than the first threshold value. The instructions, when executed by the at least one processor individually or collectively, cause the wearable device to, based on a value of sensor data related to a first designated motion among the designated motions being less than the first threshold value, identify a level of the first designated motion as a first level. The instructions, when executed by the at least one processor individually or collectively, cause the wearable device to, based on the value of the sensor data being greater than or equal to the first threshold value and less than the second threshold value, identify the level of the first designated motion as a second level. The instructions, when executed by the at least one processor individually or collectively, cause the wearable device to, based on the value of the sensor data being greater than or equal to the second threshold value, identify the level of the first designated motion as a third level.

According to example embodiment, The instructions, when executed by the at least one processor individually or collectively, cause the wearable device to display a screen for displaying results of the exercise of the user based on completion of the exercise of the user. The screen for displaying the results may represent a level related to each of the designated motions.

According to example embodiment, the exercise of the user may be performed through a plurality of sets set based on repetitions of the designated motion according to a designated value. The instructions, when executed by the at least one processor individually or collectively, cause the wearable device to identify, based on the first value corresponding to the designated value, that a first set among the plurality of sets are performed.

According to example embodiment, The instructions, when executed by the at least one processor individually or collectively, cause the wearable device to provide, based on the first value corresponding to the designated value, a notification representing end of the first set.

According to example embodiment, The instructions, when executed by the at least one processor individually or collectively, cause the wearable device to identify, based on an input for the second value, a third value indicating the number of times designated motions of a second set which is next set of the first set, among the plurality of sets, are performed, using the second threshold value.

According to example embodiment, The instructions, when executed by the at least one processor individually or collectively, cause the wearable device to display a screen representing the third value indicating the number of times the designated motions of the second set are performed based on the second threshold value, while the designated motions of the second set are being performed.

According to example embodiment, The instructions, when executed by the at least one processor individually or collectively, cause the wearable device to store a fourth value indicating the number of times the designated motions of the second set are performed based on the first threshold value, while the designated motions of the second set are being performed.

According to example embodiment, a method performed by a wearable device may comprise identifying that an exercise of a user based on designated motions is started. The method may comprise obtaining second information on the designated motions. The method may comprise obtaining second information on exercise movement of the user, in accordance with performance of the designated motions. The method may comprise obtaining, based on a first threshold value related to the first information, a first value indicating a number of times the designated motions with respect to the second information were performed. The method may comprise obtaining, based on a second threshold value related to the first information, a second value indicating the number of times the designated motions with respect to the second information were performed. The method may comprise displaying the first value and the second value on the display.

According to example embodiment, the method may comprise displaying a screen for an input of at least one of the first value and/or the second value. The screen may comprise a first visual object representing the first value and a second visual object representing the second value. The input of at least one of the first value and the second value may comprise an input for at least one of the first visual object and/or the second visual object.

According to example embodiment, the second visual object may be highlighted compared to the first visual object in the screen.

According to example embodiment, the method may comprise, based on identifying an input for the first value, maintaining a threshold value for identifying the number of times the designated motions were performed, as the first threshold value. The method may comprise, based on identifying an input for the second value, changing the threshold value for identifying the number of times the designated motions were performed, from the first threshold value to the second threshold value.

According to example embodiment, a non-transitory computer readable storage medium may store one or more programs. The one or more programs may comprise instructions which, when being executed by a at least one processor of a wearable device with a display, cause the wearable device to identify that an exercise of a user based on designated motions is started. The one or more programs may comprise instructions causing the wearable device to obtain second information on the designated motions. The one or more programs may comprise instructions causing the wearable device to obtain, based on a first threshold value related to the first information, a first value indicating a number of times the designated motions with respect to the second information were performed. The one or more programs may comprise instructions causing the wearable device to obtain, based on a second threshold value related to the first information, a second value indicating the number of times the designated motions with respect to the second information were performed. The one or more programs may comprise instructions causing the wearable device to display the first value and the second value on the display.

According to example embodiment, a wearable device may comprise a display, at least one sensor, a first memory storing a first threshold value, comprising one or more storage media, a second memory storing instructions, comprising one or more storage media, and at least one at least one processor comprising processing circuitry. The instructions, when executed by the at least one processor individually or collectively, cause the wearable device to identify that an exercise of a user based on designated motions is started. According to example embodiment, The instructions, when executed by the at least one processor individually or collectively, cause the wearable device to obtain information on the designated motions. According to example embodiment, The instructions, when executed by the at least one processor individually or collectively, cause the wearable device to obtain, based on the information, a second threshold value identifying the number of times the designated motions were performed. According to example embodiment, The instructions, when executed by the at least one processor individually or collectively, cause the wearable device to display the first threshold value and the second threshold value on the display.

According to example embodiment, The instructions, when executed by the at least one processor individually or collectively, cause the wearable device to display a screen for an input for one of the first threshold value and the second threshold value.

According to example embodiment, the screen may comprise a first visual object representing the first threshold value and a second visual object representing the second threshold value. The input for one of the first threshold value and the second threshold value may comprise an input for one of the first visual object and the second visual object.

According to example embodiment, the second visual object may be highlighted compared to the first visual object in the screen.

According to example embodiment, The instructions, when executed by the at least one processor individually or collectively, cause the wearable device to, based on identifying an input for the first threshold value, maintain a threshold value for identifying a number of times the designated motions were performed, as the first threshold value. The instructions, when executed by the at least one processor individually or collectively, cause the wearable device to, based on identifying an input for the second threshold value, change the threshold value for identifying the number of times the designated motions were performed, from the first threshold value to the second threshold value.

According to the various embodiments, a wearable device may identify an exercise of a user performed based on designated motions. The wearable device may identify the number of times the designated motions are performed. The wearable device may provide the user with the number of times the designated motions are performed. When the number of times the designated motions are performed, which is provided to the user, is different from the number of times the designated motions are actually performed, the wearable device may provide the user with a screen for correcting (or modifying) the number of times the designated motions are performed. The wearable device may increase accuracy of recognition by correcting the number of times the designated motions are performed, which is identified in the wearable device. For example, the wearable device may provide a screen for correcting the number of times counted each time the user's exercise set ends. The wearable device may display a suggested correction value in the screen so that the user may simply correct the number of times. The user may correct the number of times the designated motions are performed in a set only by inputting to an object on the screen.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, or any combination thereof, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between a case in which data is semi-permanently stored in the storage medium and a case in which the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

While the disclosure has been illustrated and described with reference to various example embodiments, it will be understood that the various example embodiments are intended to be illustrative, not limiting. It will be further understood by those skilled in the art that various changes in form and detail may be made without departing from the true spirit and full scope of the disclosure, including the appended claims and their equivalents. It will also be understood that any of the embodiment(s) described herein may be used in conjunction with any other embodiment(s) described herein.

## Claims

1. A wearable device comprising:
a display;
at least one sensor;
a first memory storing first information on exercise movement, comprising one or more storage media;
a second memory storing instructions, comprising one or more storage media; and
at least one processor, comprising processing circuitry;
wherein the instructions, when executed by the at least one processor individually or collectively, cause the wearable device to:
identify that an exercise of a user based on designated motions is started,
obtain second information on the designated motions,
obtain, based on a first threshold value related to the first information, a first value indicating a number of times the designated motions with respect to the second information were performed,
obtain, based on a second threshold value related to the first information, a second value indicating the number of times the designated motions with respect to the second information were performed, and
display the first value and the second value on the display.

2. The wearable device of claim 1, wherein the instructions, when executed by the at least one processor individually or collectively, cause the wearable device to display a screen for an input of at least one of the first value and the second value,
wherein the screen comprises a first visual object representing the first value and a second visual object representing the second value, and
wherein the input of at least one of the first value and the second value comprises an input for the first visual object or the second visual object.

3. The wearable device of claim 2, wherein the second visual object is highlighted compared to the first visual object in the screen.

4. The wearable device of claim 2, wherein the instructions, when executed by the at least one processor individually or collectively, cause the wearable device to:
based on identifying an input for the first value, maintain a threshold value for identifying the number of times the designated motions were performed, included in the first information, as the first threshold value, and
based on identifying an input for the second value, change the threshold value for identifying the number of times the designated motions were performed, included in the first information, from the first threshold value to the second threshold value.

5. The wearable device of claim 2, wherein the instructions, when executed by the at least one processor individually or collectively, cause the wearable device to:
based on identifying an input for the first value, maintain a threshold value for identifying the number of times the designated motions were performed, included in the first information, as the first threshold value, and
based on identifying an input for the second value, add the second threshold value to the threshold value for identifying the number of times the designated motions were performed, included in the first information.

6. The wearable device of claim 1, wherein the instructions, when executed by the at least one processor individually or collectively, cause the wearable device to determine the second threshold value for obtaining the second value, within a designated range.

7. The wearable device of claim 6, wherein the designated range is set based on a first reference value and a second reference value greater than the first reference value, and
wherein the instructions, when executed by the at least one processor individually or collectively, cause the wearable device to:
based on identifying that the second threshold value is less than the first reference value, determine the second threshold value as the first reference value, and
based on identifying that the second threshold value is greater than the second reference value, determine the second threshold value as the second reference value

8. The wearable device of claim 1, wherein the second threshold value is determined to be less than the first threshold value,
wherein the instructions, when executed by the at least one processor individually or collectively, cause the wearable device to:
based on a value of sensor data related to a first designated motion among the designated motions being greater than or equal to the first threshold value, identify a level of the first designated motion as a first level,
based on the value of the sensor data being less than the first threshold value and greater than or equal to the second threshold value, identify the level of the first designated motion as a second level, and
based on the value of the sensor data being less than the second threshold value, identify the level of the first designated motion as a third level.

9. The wearable device of claim 1, wherein the second threshold value is determined to be greater than the first threshold value,
wherein at least one processor, individually and/or collectively, is configured to cause the wearable device to:
based on a value of sensor data related to a first designated motion among the designated motions being less than the first threshold value, identify a level of the first designated motion as a first level,
based on the value of the sensor data being greater than or equal to the first threshold value and less than the second threshold value, identify the level of the first designated motion as a second level, and
based on the value of the sensor data being greater than or equal to the second threshold value, identify the level of the first designated motion as a third level.

10. The wearable device of claim 9, wherein the instructions, when executed by the at least one processor individually or collectively, cause the wearable device to display a screen for displaying results of the exercise of the user based on completion of the exercise of the user, and
wherein the screen for displaying the results represents a level related to each of the designated motions.

11. The wearable device of claim 2, wherein the exercise of the user is performed through a plurality of sets set based on repetitions of the designated motion according to a designated value, and
wherein the instructions, when executed by the at least one processor individually or collectively, cause the wearable device to identify, based on the first value corresponding to the designated value, that a first set among the plurality of sets are performed.

12. The wearable device of claim 11, wherein the instructions, when executed by the at least one processor individually or collectively, cause the wearable device to provide, based on the first value corresponding to the designated value, a notification representing an end of the first set.

13. The wearable device of claim 11, wherein the instructions, when executed by the at least one processor individually or collectively, cause the wearable device to identify, based on an input for the second value, a third value indicating the number of times designated motions of a second set which is next set of the first set, among the plurality of sets, are performed, using the second threshold value.

14. A method performed by a wearable device, the method comprising:
identifying that an exercise of a user based on designated motions is started,
obtaining second information on the designated motions,
obtaining, based on a first threshold value related to the first information, a first value indicating a number of times the designated motions with respect to the second information were performed,
obtaining, based on a second threshold value related to the first information, a second value indicating the number of times the designated motions with respect to the second information were performed, and
displaying the first value and the second value on the display.

15. A non-transitory computer-readable storage medium storing one or more programs, wherein the one or more programs comprise instructions which, when executed by at least one processor of a wearable device with a display, individually and/or collectively, cause the wearable device to:
identify that an exercise of a user based on designated motions is started,
obtain second information on the designated motions,
obtain, based on a first threshold value related to the first information, a first value indicating a number of times the designated motions with respect to the second information were performed,
obtain, based on a second threshold value related to the first information, a second value indicating the number of times the designated motions with respect to the second information were performed, and
display the first value and the second value on the display.
